(19) Europäisches Patentamt
European Patent Office
Office européen des brevets

(11) EP 4 782 842 A1

(12) EUROPEAN PATENT APPLICATION

(43) Date of publication:
29.07.2026 Bulletin 2026/31

(51) International Patent Classification (IPC):
*G01N 35/10* (2006.01)

(21) Application number: 26153380.6

(52) Cooperative Patent Classification (CPC):
**G01N 35/1002;** G01N 2035/1032

(22) Date of filing: 22.01.2026

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 24.01.2025 JP 2025010999

(71) Applicant: SYSMEX CORPORATION
**Kobe-shi**
**Hyogo 651-0073 (JP)**

(72) Inventors:
• NAKANISHI, Noriyuki
Kobe-shi, Hyogo, 651-0073 (JP)
• FUKUMA, Daigo
Kobe-shi, Hyogo, 651-0073 (JP)
• IKUTA, Junya
Kobe-shi, Hyogo, 651-0073 (JP)
• MISAWA, Kota
Kobe-shi, Hyogo, 651-0073 (JP)
• KUMAGAI, Atsushi
Kobe-shi, Hyogo, 651-0073 (JP)

(74) Representative: Hoffmann Eitle
Patent- und Rechtsanwälte PartmbB
Arabellastraße 30
81925 München (DE)

(54) **MEASUREMENT APPARATUS**

(57) Disclosed is a measurement apparatus for blood samples including a reagent storage unit for first and second containers storing a diluent and a hemolytic agent, respectively, and a transport unit arranged above it. A measurement unit, also above the storage unit, includes a third container for a staining solution, chambers for preparing first and second measurement samples, and detectors for electrical and optical signals. The apparatus is configured to process 300 to 500 samples per hour for measurement orders including complete blood count and five-part white blood cell differential counting. A supply unit provides reagents to the measurement unit, and a control unit manages the operations. The supply unit is configured to continue supplying the diluent and the hemolytic agent while the first or second container is being replaced, ensuring uninterrupted measurement.

FIG. 1

EP 4 782 842 A1

**Description**

CROSS REFERENCE TO RELATED APPLICATIONS

This application claims priority from prior Japanese Patent Application No. 2025-10999, filed on January 24, 2025, entitled "MEASUREMENT APPARATUS", the entire content of which is incorporated herein by reference.

FIELD OF THE INVENTION

The present invention relates to a measurement apparatus.

BACKGROUND OF THE INVENTION

International publication WO2007047069 discloses a system that supplies reagents from a reagent station, which includes containers accommodating reagents, to a plurality of reagent consumption stations. A buffer chamber is provided between the reagent station and the reagent consumption stations to store the reagents supplied from the reagent station toward the reagent consumption stations. Because reagents are stored in the buffer chamber, the supply of reagents to the reagent consumption stations can be continued even if the remaining amount of reagents in the containers of the reagent station is insufficient.

SUMMARY OF THE INVENTION

As the throughput of the reagent consumption stations improves, the reagent consumption per unit time at the reagent consumption stations increases. In a situation where the throughput of each reagent consumption station is improved, the system disclosed in WO2007047069, which is configured to supply reagents from a reagent station to a plurality of reagent consumption stations, may face a problem in which the amount of suppliable reagents becomes insufficient to meet the increased throughput of the plurality of reagent consumption stations, even though the system is provided with a buffer chamber. WO2007047069 does not address such a problem.

The present disclosure is directed to a measurement apparatus operable to measure a blood sample, comprising: a reagent storage unit configured to accommodate a first container storing a diluent to dilute a blood sample and a second container storing a hemolytic agent to lyse red blood cells contained in the blood sample; a transport unit arranged above the reagent storage unit, wherein the transport unit includes a first region and a second region on which the blood sample is placed and the transport unit is configured to transport the placed blood sample;

a measurement unit configured to measure the blood sample transported from the first region and to discharge the measured blood sample to the second region; a supply unit configured to supply the diluent and the hemolytic agent to the measurement unit; and a control unit configured to control the transport unit, the measurement unit, and the supply unit, wherein the measurement unit includes: a third container storing a staining solution to stain the blood sample; a first chamber configured for mixing the diluent supplied from the first container and the blood sample to prepare a first test sample; a second chamber configured for mixing the hemolytic agent supplied from the second container, the blood sample, and the staining solution to prepare a second test sample; and a measuring section including an electrical signal detector to interrogate the first test sample to obtain an electrical signal and an optical signal detector to interrogate the second test sample to obtain an optical signal, wherein the measurement unit is configured to obtain the electrical signal and the optical signal for 300 to 500 blood samples per hour according to a measurement order for each of the blood samples, the measurement order including: (1) a first measurement item including red blood cell count, white blood cell count, hemoglobin concentration, hematocrit value, mean corpuscular volume, mean corpuscular hemoglobin, mean corpuscular hemoglobin concentration, and platelet count; and (2) a second measurement item of five-part white blood cell differential counting, and to provide a measurement result based on the obtained electrical signal and the optical signal, and wherein the supply unit is configured to continue the supply of the diluent while the first container is being replaced and to continue the supply of the hemolytic agent while the second container is being replaced.

According to the present invention, reagent supply to the measurement unit is maintained during the replacement of the first or second container. This allows the measurement process to continue uninterrupted, even if reagent consumption increases due to enhanced throughput.

BRIEF DESCRIPTION OF THE DRAWINGS

FIG. 1 is a front view schematically showing an external appearance of the measurement apparatus according to Embodiment 1.
FIG. 2 is a plan view showing the configuration of the transport unit according to Embodiment 1.
FIG. 3 is a plan view schematically showing the configuration of the measurement unit according to Embodiment 1.
FIG. 4 is a perspective view showing an example of the configuration of the reagent storage unit according to Embodiment 1.
FIG. 5 is a diagram showing an example of a configuration for connecting the first container or the second container to the supply unit according to Embodiment 1.

FIG. 6 is a perspective view showing the configuration of a staining solution storage unit according to Embodiment 1.

FIG. 7 is a diagram schematically showing the configuration of the supply unit according to Embodiment 1.

FIG. 8 is a schematic diagram showing a configuration in which the staining solution is supplied from the third container to each chamber according to Embodiment 1.

FIG. 9 is a table showing an example of the types of reagents used by the measurement apparatus according to Embodiment 1, the capacities of each container, and the number of measurable tests.

FIG. 10 is a block diagram showing a configuration in which the test samples prepared in each chamber are supplied to the measuring section according to Embodiment 1.

FIG. 11 is a schematic diagram showing the configuration of a fluid circuit including a chamber and an optical signal detector according to Embodiment 1.

FIG. 12 is a schematic diagram showing the configuration of a fluid circuit connected to a chamber, an HGB chamber, an electrical signal detector, and an HGB measuring section according to Embodiment 1.

FIG. 13 is a diagram showing the configuration of a Flow Cell 65 of the optical signal detector 381 according to Embodiment 1.

FIG. 14 is a schematic diagram showing the configuration of a fluid circuit connected to a chamber, an HGB chamber, an electrical signal detector, and an HGB measuring section according to Embodiment 1.

FIG. 15 is a block diagram showing the functional configuration of the measurement unit according to Embodiment 1.

FIG. 16 is a block diagram showing the functional configuration of the transport unit and the analysis unit according to Embodiment 1.

FIG. 17 is a time chart of measurement by the measurement unit according to Embodiment 1.

FIG. 18 is a flowchart regarding the supply of the diluent by the measurement apparatus.

FIG. 19 is a diagram showing an example of the screen display of an alarm to prompt replacement according to Embodiment 1.

FIG. 20 is an example of the output of an alarm to prompt replacement via a notification to a terminal according to Embodiment 1.

FIG. 21 is an example of a reagent information screen according to Embodiment 1.

FIG. 22 is a flowchart showing an example of the supply of staining solution according to a modified example of Embodiment 1.

FIG. 23 is a diagram showing the configuration of the supply unit according to Embodiment 2.

FIG. 24 is an example of a reagent information screen according to Embodiment 2.

FIG. 25 is a diagram showing an example of identification information attached to a container accommodated in the reagent storage unit according to Embodiment 2.

FIG. 26 is a flowchart regarding the automatically switch of reagents according to Embodiment 24.

FIG. 27 is a schematic diagram showing the configuration of the reagent supply unit according to Embodiment 3.

FIG. 28 is a flowchart showing an example of the supply of the diluent according to Embodiment 3.

FIG. 29 is a schematic diagram showing the configuration of the reagent supply unit according to Embodiment 4.

FIG. 30 is a schematic diagram showing the configuration of the reagent supply unit according to Embodiment 5.

FIG. 31 is a modified example of the reagent information screen.

DETAILED DESCRIPTION

The measurement apparatus of Embodiment 1 is a measurement apparatus that measures a blood sample using a diluent and a hemolytic agent. The diluent is accommodated in a first container, and the hemolytic agent is accommodated in a second container. The measurement unit performs measurement by receiving the supply of the diluent and the hemolytic agent from the first container and the second container. The processing speed of the measurement unit is, for example, 300 to 500 blood samples per hour. This measurement unit realizes continuous loading by the first container and the second container respectively connected to reserve tanks, and continuing the supply of the diluent and the hemolytic agent from the respective reserve tanks while the first container or the second container is being replaced. The measurement apparatus of Embodiment 2 is configured such that first and fourth containers storing the diluent are switchably connected, and second and fifth containers storing the hemolytic agent are switchably connected. Continuous loading is realized by continuing the supply of the diluent from the fourth container when the supply of the diluent from the first container becomes impossible, and continuing the supply of the hemolytic agent from the fifth container when the supply of the hemolytic agent from the second container becomes impossible. The measurement apparatus of Embodiment 3 is a measurement apparatus that is equipped with the reserve tank provided in the measurement apparatus of Embodiment 1 and the switching function provided in the measurement apparatus of Embodiment 2. The measurement apparatus of Embodiment 4 realizes Continuous Loading by diluting the Concentrated Diluent accommodated in the first container at a predetermined dilution ratio and supplying it to the measurement unit, and continuing the supply of the diluent from a Dilution Tank while the first container is being replaced. The measurement apparatus of Embodiment 5 is a measurement apparatus that is equipped with a switching function for the Concentrated Diluent in addition to the Dilution Tank provided in the measurement apparatus of Embodiment 4.

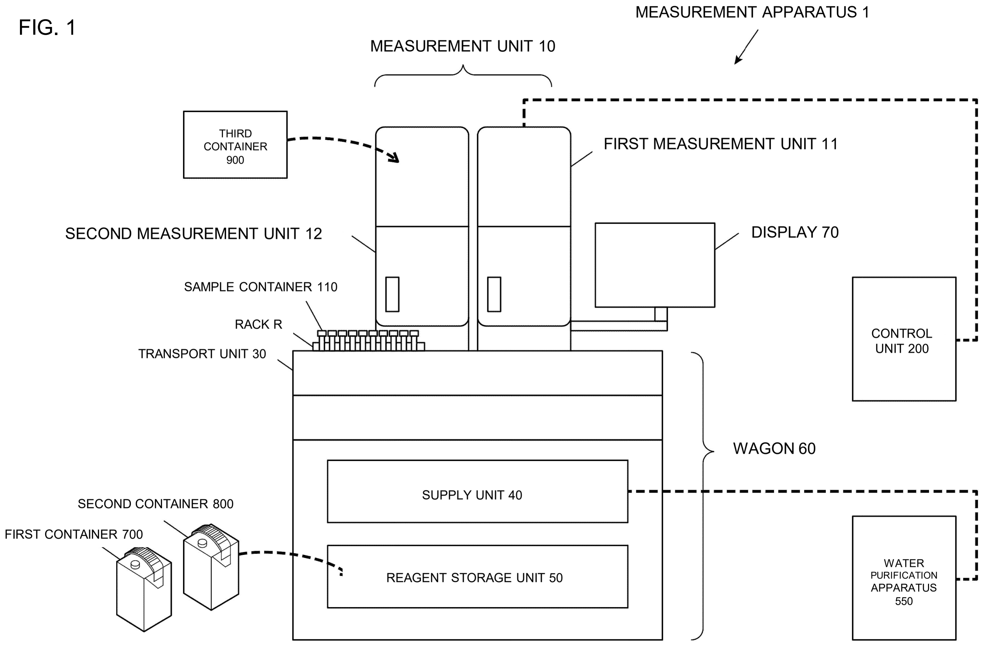

<Embodiment 1> FIG. 1 is a front view schematically showing a configuration example of the measurement apparatus 1.

The measurement apparatus 1 includes, for example, a measurement unit 10, a transport unit 30, a supply unit 40, a reagent storage unit 50, and a control unit 200.

The measurement apparatus 1 is, for example, an apparatus that measures a blood sample. The measurement apparatus 1 is, for example, a blood cell counting apparatus that counts blood cells contained in whole blood by measuring whole blood. The measurement apparatus 1 is, for example, connected to a Host Computer HC, and measures a blood sample based on a measurement order input to the Host Computer HC. The measurement order may be directly input to the measurement apparatus 1. The measurement apparatus 1 may acquire the measurement order from a computer other than the Host Computer HC. The measurement order includes, for example: (1) measurement instructions for a first measurement item including red blood cell count, white blood cell count, hemoglobin concentration, hematocrit value, mean corpuscular volume, mean corpuscular hemoglobin, mean corpuscular hemoglobin concentration, and platelet count; and (2) measurement instructions for a second measurement item of five-part white blood cell differential counting. The measurement apparatus 1 is capable of measuring, for example, 300 to 500 blood samples per hour for the measurement order of the first measurement item and the second measurement item.

The measurement apparatus 1 illustrated in FIG. 1 has "one-to-one" relationship, rather than "one-to-many" relationship, between the supply unit 40 as the reagent supply source and the measurement unit 10 as the reagent supply destination. The measurement apparatus 1 is configured to operate at a predetermined throughput (in the example of this embodiment, 300 to 500 blood samples per hour) as a standalone apparatus. The supply unit 40 can perform continuous loading of reagents within the measurement apparatus 1 that processes blood samples with the predetermined throughput as a standalone apparatus. The supply unit 40 is exclusively used for the corresponding measurement unit 10 and supplies reagents to the corresponding measurement unit 10 via a flow path to exclusively supply the reagents. With such a configuration of the measurement apparatus 1, the supply unit 40 for continuously supplying reagents can be simple design depending on the throughput of the measurement unit 10 that consumes the reagents. When the system is expanded, for example, a plurality of measurement apparatuses 1 are installed, and each measurement apparatus 1 is connected by a transport system capable of transporting blood samples or racks accommodating the blood samples therebetween. Even if a plurality of measurement apparatuses 1 are installed due to such a system expansion, continuous loading of reagents can be performed within each compartment of measurement apparatus 1. It means that each of the plurality of measurement apparatuses 1 is a module in the expanded system. The system is expanded by combining the measurement apparatuses 1 as modules. Therefore, even if the system is expanded, the capacity of reagent supply of the supply unit 40 only needs to be designed solely on the throughput of the individual measurement apparatus 1, enabling the design of reagent continuous loading independently of the throughput of the overall expanded system.

The measurement unit 10 includes, for example, a first measurement unit 11 and a second measurement unit 12. The measurement unit 10 may be configured only by either the first measurement unit 11 or the second measurement unit 12. For example, the two measurement units are arranged adjacently. The first measurement unit 11 and the second measurement unit 12 can perform blood sample measurement in parallel. For example, when a blood sample (A) and a blood sample (B) are supplied to the measurement apparatus 1, the first measurement unit 11 measures the blood sample (A), and in parallel with this, the second measurement unit 12 can measure the blood sample (B). By such an operation, the overall throughput of the measurement apparatus 1 is improved. The transport unit 30, for example, transports a rack R holding a sample container 110 to distribute a plurality of blood samples to the first measurement unit 11 and the second measurement unit 12. The measurement unit 10 and the transport unit 30 are provided on a wagon 60.

The reagent storage unit 50 is provided inside the wagon 60. The reagent storage unit 50 accommodates a first container 700 storing a diluent and a second container 800 storing a hemolytic agent.

The supply unit 40 is accommodated inside the wagon 60. The supply unit 40 supplies the diluent from the first container 700 accommodated in the reagent storage unit 50 to the measurement unit 10, and supplies the hemolytic agent from the second container 800 accommodated in the reagent storage unit 50 to the measurement unit 10. The measurement unit 10 aspirates the blood sample accommodated in the sample container 110 transported by the transport unit 30. The measurement unit 10 mixes the aspirated blood sample and reagents to prepare a measurement sample, and measures the prepared measurement sample. The reagents mixed with the blood sample are the diluent and the hemolytic agent supplied by the supply unit 40, and the staining solution accommodated in the third container 900 installed in the measurement unit 10.

In one example, a display 70 is connected to the measurement unit 10. The display 70 is connected to the measurement unit 10 via, for example, a movably configured arm. The display 70, for example, displays an operation screen as an interface for operating the measurement apparatus 1, and displays the analysis results obtained by the measurement apparatus 1 measuring the blood sample.

The control unit 200 controls the operation of each operable units (measurement unit 10, transport unit 30, supply unit 40, etc.) for the measurement apparatus 1 to measure the blood sample, analyzes the measurement data obtained by the measurement unit 10 measuring the blood sample to generate an analysis result, and provides the generated analysis result.

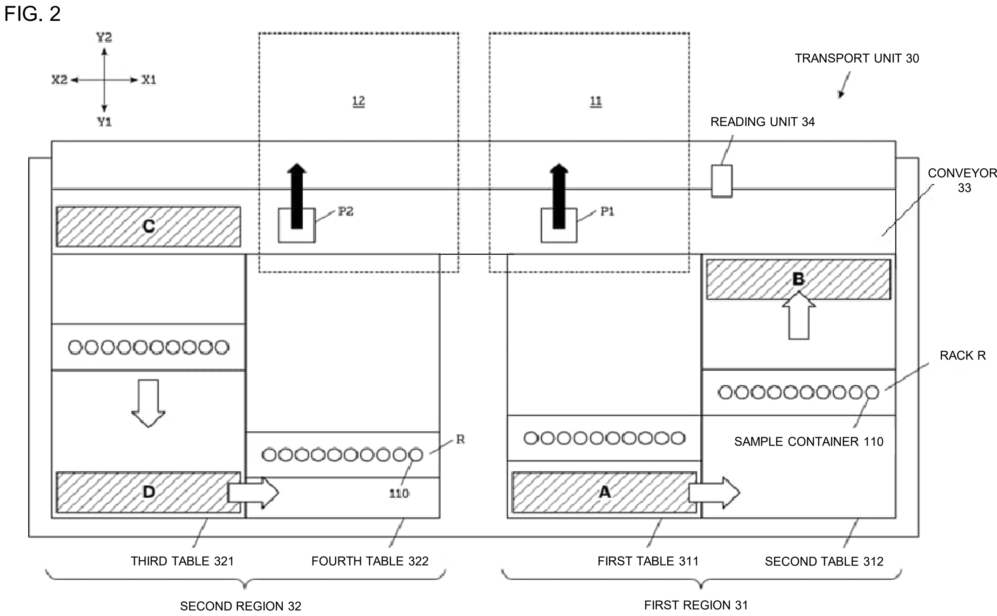

FIG. 2 is a plan view showing a configuration example of the transport unit 30. The transport unit 30 includes a first region 31 on which a rack R holding a pre-measurement blood sample that is to be measured by the measurement unit 10 is placed, and a second region 32 on which a rack R holding a measured blood sample is placed.

The first region 31 includes a first table 311 and a second table 312. The first table 311 supports the placed rack R and transports the rack R in the Y1 direction. The rack R transported to the position indicated by A in FIG. 2 is transported in the X1 direction and moves to the second table 312. The second table 312 transports the rack R received from the first table 311 in the Y2 direction. The rack R transported to the position indicated by B in FIG. 2 is further transported in the Y2 direction and moves onto a conveyor 33.

The conveyor 33 transports the rack R in the X2 direction, and transports the sample container 110 mounted on the rack R to an access position P1 of the first measurement unit 11 and an access position P2 of the second measurement unit 12, thereby supplying the sample container 110 to the first measurement unit 11 or the second measurement unit 12. The first measurement unit 11 accesses the sample container 110 positioned at P1 and aspirates the blood sample accommodated in the sample container 110. The second measurement unit 12 accesses the sample container 110 positioned at P2 and aspirates the blood sample accommodated in the sample container 110. The conveyor 33 transports the rack R in the X2 direction after the supply of the sample container 110 to the first measurement unit 11 or the second measurement unit 12. The conveyor 33 transports the rack R to a position adjacent to the second region 32 (the position indicated by C in FIG. 2).

The second region 32 includes a third table 321 and a fourth table 322. The third table 321 receives the rack R moved from the conveyor 33 and transports the rack R in the Y1 direction. The rack R transported to the position indicated by D in FIG. 2 is transported in the X1 direction and moves to the fourth table 322. The fourth table 322 transports the rack R received from the third table 321 in the Y2 direction. As a result, the rack R for which the supply to the measurement unit 10 has been completed is lined up from the rear (Y2 direction side) of the fourth table 322. The rack R lined up on the fourth table 322 is taken out by the user.

The transport unit 30 includes a reading unit 34 at a position adjacent to the conveyor 33. The reading unit 34 is a code reader that reads a machine-readable code affixed to the sample container 110 held in the rack R. By the reading unit 34 reading the code, the blood sample ID of the blood sample accommodated in the sample container 110 is acquired.

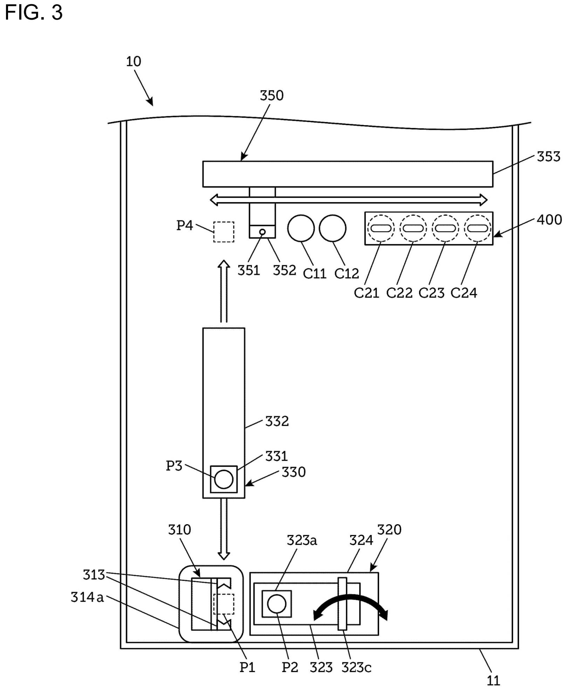

FIG. 3 is a plan view schematically showing a configuration example of the measurement unit 10.

The measurement unit 10 includes a gripping mechanism 310, an agitator 320, a container transfer mechanism 330, a dispensing mechanism 350, an RBC/PLT chamber C11, an HGB chamber C12, and a chamber heating unit 400.

The gripping mechanism 310 includes a pair of gripping pieces 313 for gripping the sample container 110 from the front-rear direction, a mechanism for moving the pair of gripping pieces 313 toward and away from each other, and a mechanism for moving the pair of gripping pieces 313 in the vertical direction. The gripping mechanism 310 is arranged above the access position P1 on the conveyor 33 (see FIG. 2). FIG. 3 illustrates the first measurement unit 11. The gripping mechanism 310 of the second measurement unit 12 is arranged above the access position P2. An opening 314a is formed in the lower surface of the housing 314 so that the pair of gripping pieces 313 can move vertically with respect to the lower surface of the housing 314.

The gripping mechanism 310 grips the sample container 110 positioned at the access position P1 using the pair of gripping pieces 313, and moves the pair of gripping pieces 313 upward to remove the sample container 110 from the rack R and lift the removed sample container 110 above the access position P1.

The agitator 320 includes a holding section 323, a slide section 324, and a mechanism for driving the holding section 323 and the slide section 324. The holding section 323 includes a holding member 323a formed with a hole capable of holding the sample container 110, and a rotation shaft 323c extending in the front-rear direction. The slide section 324 supports the lower part of the holding section 323 and is configured to be movable in the left-right direction. The holding section 323 is configured to be rotatable around the rotation shaft 323c.

The agitation of the sample container 110 is performed as follows. The gripping mechanism 310 grips the sample container 110 at the access position P1, takes the gripped sample container 110 out from the rack R, and transfers it upward. The agitator 320 moves the slide section 324 to the left and positions the holding member 323a below the lifted sample container 110. In this state, the gripping mechanism 310 places the sample container 110 in the holding member 323a by moving the gripped sample container 110 downward. The gripping mechanism 310 releases the sample container 110. The agitator 320 transfers the slide section 324 to the right and positions the sample container 110 held by the holding member 323a at the agitation position. When the agitator 320 rotates the holding section 323 around the rotation shaft 323c, the sample container 110 held by the holding member 323a is inverted upside down. When the agitator 320 rotates the holding section 323 in the reverse direction, the sample container 110 is returned to its original orientation. By repeating this forward and reverse rotation, the sample container 110 is turned over and agitated multiple times, and the blood sample is agitated.

The container transfer mechanism 330 includes a holding member 331 formed with a hole capable of holding the sample container 110, a plate member 332 supporting the holding member 331 and extending in the front-rear direction, and a mechanism for moving the plate member 332 in the front-rear direction.

After the agitation of the sample container 110 by the agitator 320 is completed, the agitator 320 repositions the sample container 110 held by the holding member 323a above the takeout position P1. The gripping mechanism 310 grips the sample container 110 held by the holding member 323a and takes it out upward. In this state, the agitator 320 retracts the holding section 323 and the slide section 324 to the right. The container transfer mechanism 330 positions the holding member 331 below the sample container 110. The gripping mechanism 310 sets the sample container 110 in the holding member 331 by moving the gripped sample container 110 downward. The gripping mechanism 310 releases the sample container 110.

After the sample container 110 is placed in the holding member 331, the container transfer mechanism 330 moves backward, and the sample container 110 is positioned at the aspiration position by the dispensing mechanism 350.

The dispensing mechanism 350 includes an aspiration pipette 351 having a high-rigidity and extending in the vertical direction, and a transfer unit 353 that transfers the aspiration pipette 351 in the vertical direction and left-right direction. The tip of the aspiration pipette 351 is formed sharply so that it can pierce (penetrate) the cap closing the upper opening of the sample container 110.

After the sample container 110 is positioned at the aspiration position, the dispensing mechanism 350 drives the transfer unit 353 to move the aspiration pipette 351 downward, piercing the cap of the sample container 110 with the tip of the aspiration pipette 351. The tip of the aspiration pipette 351 reaches a depth at a predetermined distance from the inner bottom of the sample container 110. In this state, the dispensing mechanism 350 aspirates the blood sample in the sample container 110 via the aspiration pipette 351.

The chamber heating unit 400 includes chamber C21 to C24. The chambers are arranged adjacently. The detailed configuration of the chamber heating unit 400 will be described later.

The dispensing mechanism 350 dispenses the blood sample aspirated at the aspiration position P4 via the aspiration pipette 351 into at least one of the chambers C11, C12, and C21 to C24 based on the measurement order. In each chamber C11, C12, and C21 to C24, the blood sample and the reagents are mixed, and a measurement sample is prepared.

After the aspiration of the blood sample from the sample container 110 is finished, the container transfer mechanism 330 positions the sample container 110 held by the holding member 331 above the access position P1, and the gripping mechanism 310 grips the sample container 110 from the holding member 331 and lifts it upward. In this state, the container transfer mechanism 330 retracts the holding member 331 backward. The gripping mechanism 310 returns the gripped sample container 110 to the original hole in the rack R.

As described, the measurement unit 10 includes the gripping mechanism 310 to remove the sample container 110 transported by the transport unit 30 from the rack R, and the agitator 320 that agitates the removed sample container 110, as separate mechanisms. This allows the operation of the gripping mechanism 310 taking out the sample container 110 from the rack R or returning the sample container 110 to the rack R, and the operation of the agitator 320 agitating the sample container 110 to be performed in parallel. This configuration contributes to increase of the throughput of the measurement unit 10.

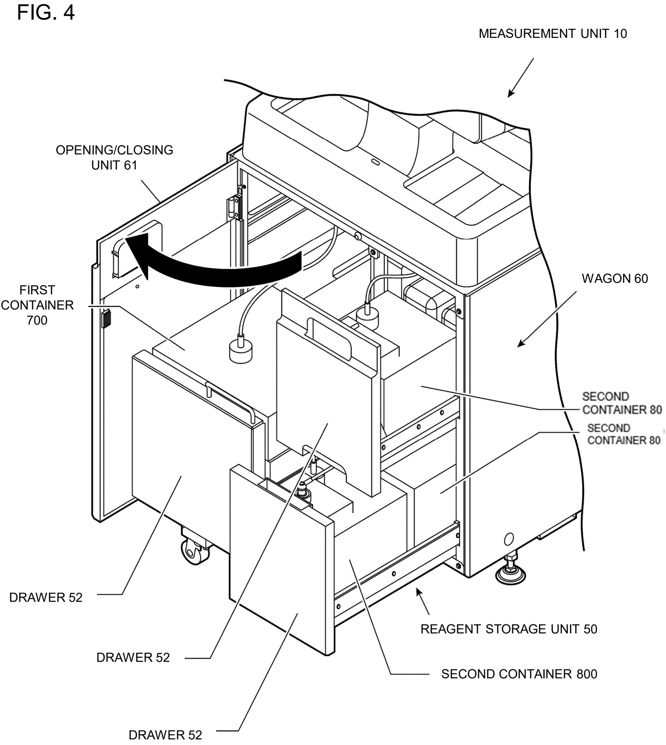

FIG. 4 is a perspective view showing an example of a configuration example of the reagent storage unit 50. As shown in FIG. 4, the wagon 60 includes an openable and closable section 61 on the front surface. The openable and closable section 61 is a door that can be pulled forward. By opening the openable and closable section 61, a user can access the reagent storage unit 50. The reagent storage unit 50 includes a plurality of drawers 52. One or more reagent containers are placed on the drawer 52. When a user wants to replace a reagent, for example, the user can pull out the drawer 52 on which the target reagent container is placed, as illustrated in FIG. 4. In the example of FIG. 4, the first container 700 storing the diluent is placed on the left drawer 52, and the second container 800 storing the hemolytic agent is placed on the right drawer 52. The wagon 60 and the reagent storage unit 50 are exclusively used for the measurement unit 10 installed on the upper surface of the wagon 60. The wagon 60 and the reagent storage unit 50 are dedicated to the measurement unit 10 installed on the upper surface of the wagon 60, supplying the reagents from the containers exclusively to the measurement unit 10 via a flow path.

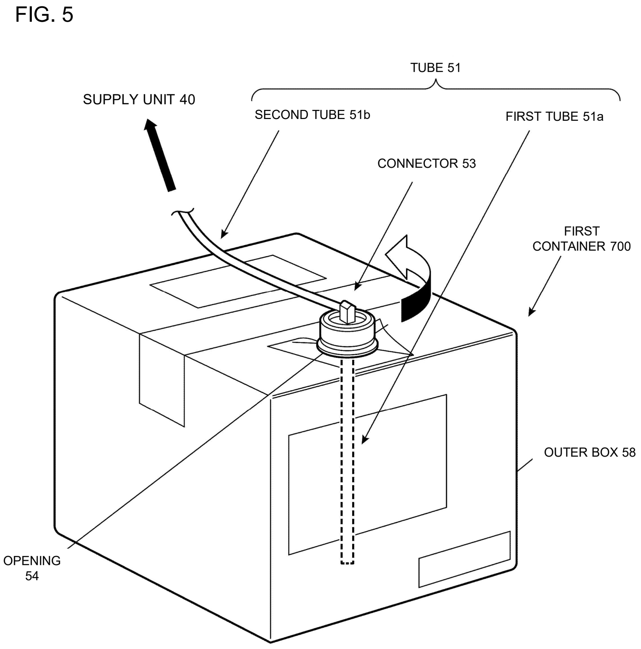

FIG. 5 is a diagram showing a configuration example for connecting the first container 700 or the second container 800 to the supply unit 40. Although the first container 700 is illustrated in FIG. 4, the connection with the supply unit 40 for the second container 800 is also the same. The supply unit 40 includes a tube 51 that is inserted into the container and delivers the reagent in the container. The tube includes a first tube 51a inserted into the container, a second tube 51b connected to the first tube 51a, and a connector 53 provided between the first tube 51a and the second tube 51b.

The first container 700 includes an outer box 58. An opening 54, which forms an opening into which the first tube 51a is inserted, is provided on the upper part of the outer box 58. The opening 54 has a screw-type cylinder that can be opened and closed by a screw cap. The opening 54 is closed by a screw cap during the distribution of the first container 700; when used, the screw cap is removed, the opening 54 is opened, and the first tube 51a is inserted through the opening 54. The connector 53 has the shape of a screw cap, and the first tube 51a is supported so as to hang down from the center of the cap. By fitting the connector 53 into the opening 54, the tip of the first tube 51a is fixed in contact with the inner bottom of the first container 700.

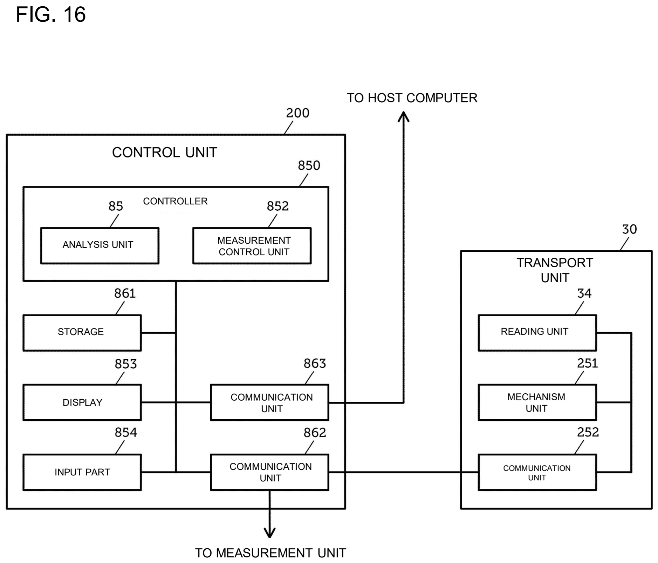

The first tube 51a is connected to the second tube 51b via the connector 53. The second tube 51b is connected to a reserve tank RT of the supply unit 40, which will be described later. The reserve tank RT is depressurized by a negative pressure generated by a pump 57. When the reserve tank RT is depressurized, the diluent in the first container 700 is aspirated from the tip of the first tube 51a. In this way, the supply unit 40 supplies the diluent accommodated in the first container 700 to the reserve tank RT by operating the pump 57 under control of the controller 852 (see FIG. 16). The second container 800 has the same configuration as the first container 700, and the hemolytic agent of the second container 800 is supplied to the reserve tank RT via the first tube 51a and the second tube 51b.

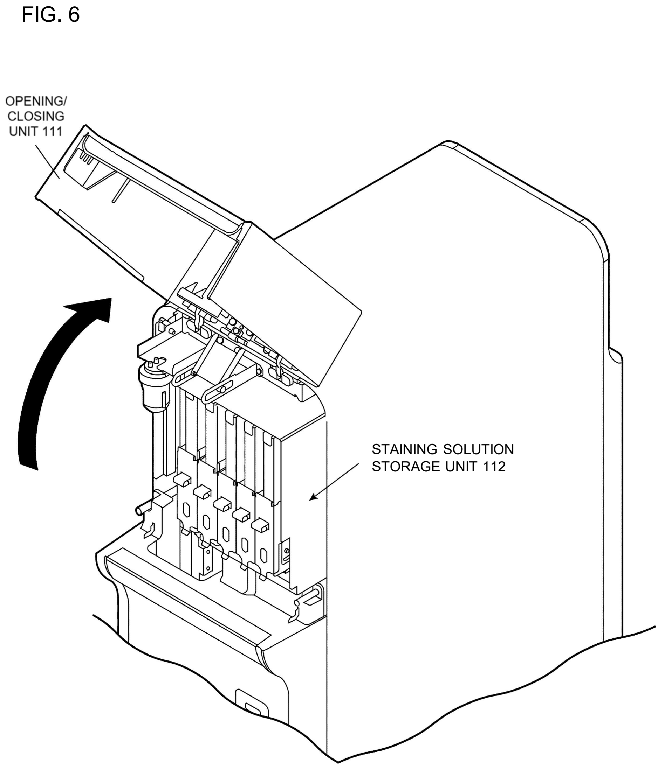

FIG. 6 is a perspective view showing a configuration example of a staining solution storage unit 112. The measurement unit 10 includes an openable and closable section 111 on the front surface. The openable and closable section 111 is a part of the front cover of the measurement unit 10 and can be opened by pushing it up. When the openable and closable section 111 is opened, the staining solution storage unit 112 is exposed. The staining solution storage unit 112 can accommodate third containers each storing the staining solution. The third container is, for example, a container molded from plastic. The third container may be a pouch container made of materials such as aluminum, polypropylene, or PET (Polyethylene Terephthalate).

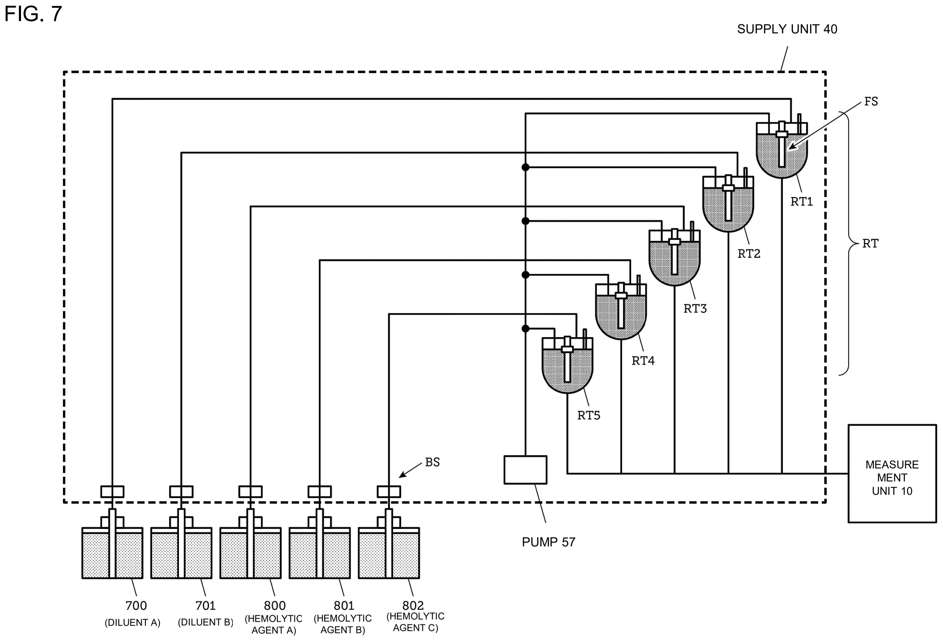

FIG. 7 is a diagram schematically showing the configuration of the supply unit 40. The measurement apparatus 1 uses five types of reagents, including two types of diluent and three types of hemolytic agent. The diluents include a diluent A for diluting blood samples for the measurement of red blood cells and/or platelet count by the electrical signal detector 382, and a diluent B for diluting blood samples for the measurement of red blood cells and/or platelet count by the optical signal detector 381. The hemolytic agents include a hemolytic agent A used for five-part white blood cell differential counting by the optical signal detector 381, a hemolytic agent B used for white blood cell count by the optical signal detector 381, and a hemolytic agent C used for hemoglobin concentration measurement by the HGB measuring section 383. The diluent A and the diluent B are respectively accommodated in the first containers 700 and 701. The hemolytic agents A, B, and C are respectively accommodated in the second containers 800, 801, and 802. These five containers are accommodated in the reagent storage unit 50, and each container is connected to the supply unit 40 via the tube 51.

The supply unit 40 includes, for example, five reserve tanks RT. The first container 700 storing the diluent A is connected to the measurement unit 10 via a first reserve tank RT1. The first container 701 storing the diluent B is connected to the measurement unit 10 via a second reserve tank RT2. The second container 800 storing the hemolytic agent A is connected to the measurement unit 10 via a reserve tank RT3. The second container 801 storing the hemolytic agent B is connected to the measurement unit 10 via a reserve tank RT4. The second container 802 storing the hemolytic agent C is connected to the measurement unit 10 via a reserve tank RT5. The flow paths connecting each container and reserve tank to the measurement unit 10 are used exclusively for the measurement unit 10 corresponding to the supply unit 40. The supply unit 40 supplies reagents to the corresponding measurement unit 10 via the flow paths connecting each container and reserve tank to the measurement unit 10, utilizing a flow path to exclusively supply the reagents.

The first container 700 and the first reserve tank RT1 are connected via the tube described with reference to FIG. 5. The first reserve tank RT1 and the measurement unit 10 are connected via a tube, and the diluent is supplied to the measurement unit 10 by drawing the diluent from the bottom of the first reserve tank RT1. The other first container 701 and second containers 800 to 802 are also connected similarly to the corresponding reserve tanks RT.

The reserve tanks RT1 to RT5 are each provided with a float sensor FS. The float sensor FS detects the remaining amount of reagent in each reserve tank. For example, when the float sensor FS of the first reserve tank RT1 detects a decrease in the reagent remaining amount, the supply unit 40 drives the pump 57 by the controller 852 (see FIG. 16) to supply the diluent A accommodated in the first container 700 to the first reserve tank RT1. Similarly for the other reserve tanks, reagent is replenished from the corresponding containers to the reserve tank RT based on the signal from the float sensor FS.

The supply unit 40 includes, for example, a bubble sensor BS provided in the flow path connecting each container and the reserve tank RT. The bubble sensor BS detects bubbles entering the flow path when the pump 57 aspirates the reagent after the reagent in the container has been exhausted. When the bubble sensor BS detects bubbles, the system detects that the corresponding container is empty.

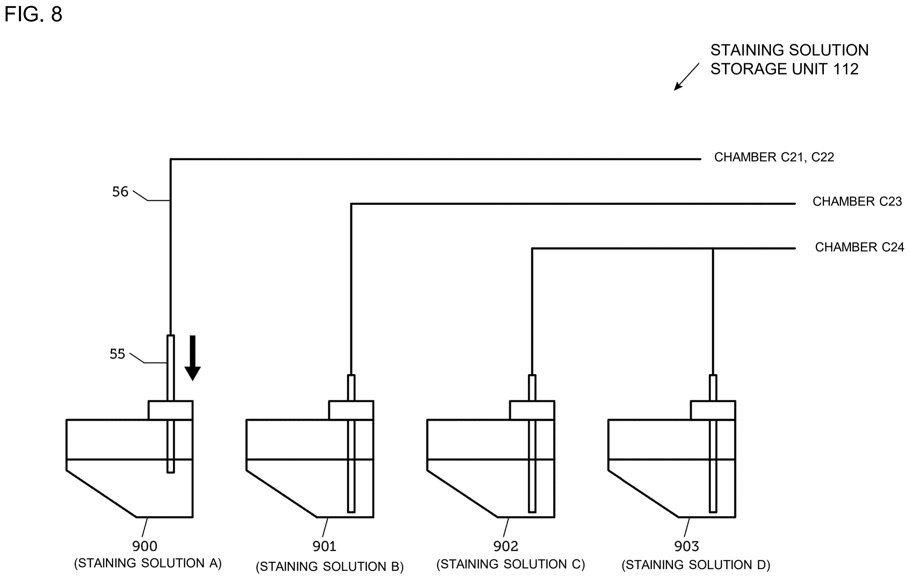

FIG. 8 is a schematic diagram showing a configuration example in which the staining solution is supplied from the third container set in the staining solution storage unit 112 to each chamber. The measurement apparatus 1 uses four types of staining solution. The staining solutions include a staining solution A used for five-part white blood cell differential counting, a staining solution B used for white blood cell count, a staining solution C used for reticulocyte measurement, and a staining solution D used for optical measurement of platelet count. The staining solutions A to D are respectively accommodated in the third containers 900, 901, 902, and 903. The third container is, for example, a cartridge-type container formed of resin, and is inserted from the front into a plurality of partitioned holders provided in the staining solution storage unit 112. The third container is loaded into the measurement apparatus 1 by inserting an aspiration pipette 55 into the interior of the third container.

The third container 900 storing the staining solution A is connected to chambers C21 and C22. The measurement unit 10 aspirates the staining solution A via the aspiration pipette 55 inserted into the third container 900, and supplies the staining solution A to the chambers C21 and C22 via a liquid delivery tube 56. The third container 901 storing the staining solution B is connected to the chamber C23. The third container 902 storing the staining solution C and the third container 903 storing the staining solution D are connected to the chamber C24. The staining solutions B to D are also supplied to the corresponding chambers similarly to the staining solution A.

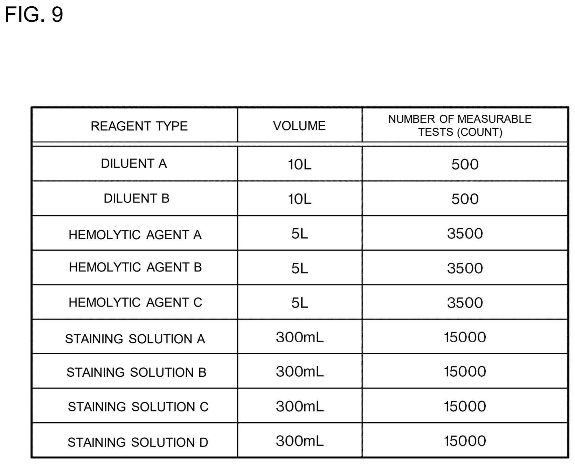

FIG. 9 is a table showing an example of the types of reagents used by the measurement apparatus 1, the capacity of each container, and the number of measurable tests. The first container storing the diluent A accommodates 10 L of the diluent A. The number of measurable tests using 10 L of the diluent A is 500 tests. The first container storing the diluent B accommodates 10 L of the diluent B. The number of measurable tests using 10 L of the diluent B is 500 tests. The second container storing the hemolytic agent A accommodates 5 L of the hemolytic agent A. The number of measurable tests using 5 L of the hemolytic agent A is 3500 tests. The second container storing the hemolytic agent B accommodates 5 L of the hemolytic agent B. The number of measurable tests using 5 L of the hemolytic agent B is 3500 tests. The second container storing the hemolytic agent C accommodates 5 L of the hemolytic agent C. The number of measurable tests using 5 L of the hemolytic agent C is 3500 tests. The third container storing the staining solution A accommodates 300 mL of the staining solution A. The number of measurable tests using 300 mL of the staining solution A is 15000 tests. The third container storing the staining solution B accommodates 300 mL of the staining solution B. The number of measurable tests using 300 mL of the staining solution B is 15000 tests. The third container storing the staining solution C accommodates 300 mL of the staining solution C. The number of measurable tests using 300 mL of the staining solution C is 15000 tests. The third container storing the staining solution D accommodates 300 mL of the staining solution D. The number of measurable tests using 300 mL of the staining solution D is 15000 tests.

The capacities of the respective reagents shown in FIG. 9 are merely examples and can be changed as appropriate. For example, if the amount of diluent solutions A and B housed in the first container is 6 L, 300 tests can be measured. If the amount of diluent solutions A and B in the first container is 30 L, 1500 tests can be measured. If the measurable number of tests falls below 300, it cannot sustain one hour of operation in a measuring apparatus with a throughput of 300 samples per hour. Therefore, it is preferable for the first container to accommodate an amount of diluent solution corresponding to at least one hour of throughput. On the other hand, increasing the capacity of the first container to reduce the frequency of replacement makes the container heavy, increasing the user's physical burden associated with reagent replacement. Therefore, the amount of diluent solutions housed in the first container is preferably 30 L (approx. 30 kg) or less. Accordingly, it is preferable that the amount of diluent solutions A and B housed in the first container corresponds to a measurable number of tests of 300 or more and 1500 or less.

Similarly, the amount of hemolytic agents A, B, and C housed in the second container may correspond to a number of tests of 2100 or more and 7000 or less. For example, if the amount of hemolytic agents A, B, and C is 3 L, 2100 tests can be measured. If the amount of hemolytic agents A, B, and C housed in the second container is, for example, 20 L, 7000 tests can be measured. The amount of staining solutions A, B, C, and D housed in the third container may correspond to a number of tests of 5000 or more and 30000 or less. For example, if the amount of staining solutions A, B, C, and D is 100 mL, 5000 tests can be measured. If the amount of staining solutions A, B, C, and D is 600 mL, 30000 tests can be measured.

As illustrated in FIG. 9, the measurable number of samples for staining solutions A, B, C, and D housed in the third container is greater than the measurable number of samples for diluent solutions A and B housed in the first container. Furthermore, the measurable number of samples for staining solutions A, B, C, and D housed in the third container is greater than the measurable number of samples for hemolytic agents A, B, and C housed in the second container. The amount of diluent solution and hemolytic agent used per measurement is greater than that of the staining solution. Therefore, while the capacity of the diluent solution and the hemolytic agent is greater than that of the staining solution, the measurable number of tests per container for the diluent solution and the hemolytic agent is less than that of the staining solution. Consequently, the replacement frequency of the first and second containers is higher than the replacement frequency of the staining solution container. The amount of staining solution housed in the third container used per measurement is less than that of the diluent solution and the hemolytic agent. Therefore, while the capacity of the staining solution is less than that of the diluent solution and the hemolytic agent, the measurable number of tests per container for the staining solution is greater than that of the diluent solution and the hemolytic agent. The frequency of replacing the third container is less than that of the first and second containers. Therefore, reserve tanks are provided for the frequently replaced diluent solution and hemolytic agent to achieve continuous loading, while the staining solution, which is replaced less frequently, is not provided with a reserve tank, allowing for the downsizing of the measurement unit.

The capacity of the first reserve tanks 710 and 711 may be smaller than that of the first containers 700 and 701. The capacity of the first reserve tank may be determined based on the number of samples measurable using the diluent reserved in the first reserve tank, the duration for which the supply of the reserved diluent can be continued, or the relationship with the capacity of the first container. If the capacity is determined based on the measurable number of samples, the capacity may be, for example, an amount sufficient to measure 300, 200, 100, 50, or 20 blood samples. If the capacity is determined based on the supply duration, the amount may be, for example, 6 L (allowing for one hour of continuous supply, assuming 300 blood samples are measured per hour using 6 L of diluent), 3 L (30 minutes), 1.5 L (15 minutes), or 0.5 L (5 minutes). If the capacity is determined based on the relationship with the capacity of the first container, the amount may be, for example, 9 L (90% of the capacity of the first container), 8 L (80%), 7 L (70%), 6 L (60%), 5 L (50%), 4 L (40%), 3 L (30%), 2 L (20%), or 1 L (10%). The capacity of the second reserve tank may be determined in the same manner.

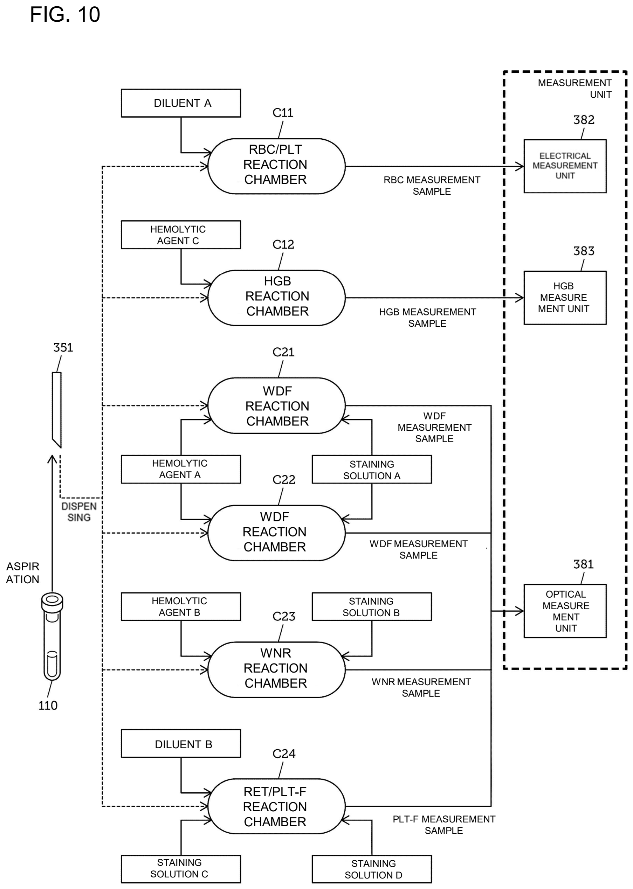

FIG. 10 is a block diagram showing an example of a configuration in which measurement samples prepared in respective chambers C11, C12, and C21 to C24 provided in the measurement unit 10 are supplied to the optical measurement unit 381, the electrical measurement unit 382, and the HGB measurement unit 383.

The operation described with reference to FIG. 10 is performed by the measurement control unit 852 (see FIG. 16) of the control unit 200, which controls the dispensing mechanism 350, the preparation of measurement samples in the respective chambers C11, C12, and C21 to C24, and the measurement by the respective measurement units 381 to 383.

Chamber C11 is a chamber for preparing a sample for the measurement of red blood cells and platelets by the electrical measurement unit 382. Diluent solution A housed in the first container 700 is supplied to the chamber C11 via the reserve tank RT1 (see FIG. 8). Diluent solution A and the sample are mixed to prepare an RBC/PLT measurement sample in which the blood sample is diluted. The RBC/PLT measurement sample prepared in the chamber C11 is measured by the electrical measurement unit 382. The electrical measurement unit 382 interrogates the cellular components in the RBC/PLT measurement sample to obtain electrical signals corresponding to red blood cells and platelets. The analysis unit 851 (see FIG. 16) of the control unit 200 analyzes the measurement results of the RBC/PLT measurement sample to obtain the red blood cell count, platelet count, and the like.

Chamber C12 is a chamber for preparing a sample for measuring the hemoglobin concentration in the HGB measurement unit 383. Hemolytic agent C housed in the second container 802 is supplied to the chamber C12. Hemolytic agent C and the sample are mixed to prepare an HGB measurement sample in which red blood cells are lysed and hemoglobin is transformed to SLS-hemoglobin. The HGB measurement sample prepared in the chamber C12 is measured by the HGB measurement unit 383. The HGB measurement unit 383 interrogates the HGB measurement sample to obtain absorbance corresponding to the hemoglobin concentration. The analysis unit 851 of the control unit 200 analyzes the absorbance to obtain the hemoglobin concentration and the like.

Chamber C21 is a chamber for preparing a sample for leukocyte classification by the optical measurement unit 381. Hemolytic agent A housed in the second container 800 is supplied to the chamber C21 via the reserve tank RT3 (see FIG. 8), and staining solution A housed in the third container 900 is also supplied. Hemolytic agent A, staining solution A, and the sample are mixed to prepare a WDF measurement sample in which the red blood cells are lysed and white blood cells are stained. The WDF measurement sample prepared in the chamber C21 is measured by the optical measurement unit 381. The optical measurement unit 381 interrogates the WDF measurement sample to obtain optical signals corresponding to white blood cells (leukocytes). The analysis unit 851 of the control unit 200 analyzes the measurement results of the WDF measurement sample prepared in the WDF chamber C21 and performs classification of leukocytes (e.g., 5-part classification: neutrophils, lymphocytes, monocytes, eosinophils, and basophils) and the like. Chamber C22 has the same function as chamber C21.

Chamber C23 is a chamber for preparing a sample for leukocyte counting by the optical measurement unit 381. Hemolytic agent B housed in the second container 801 is supplied to the chamber C23 via the reserve tank RT4 (see FIG. 8), and staining solution B housed in the third container 901 is also supplied. Hemolytic agent B, staining solution B, and the sample are mixed to prepare a WNR measurement sample in which the red blood cells are lysed and white blood cells are stained. The WNR measurement sample prepared in the chamber C23 is measured by the optical measurement unit 381. The optical measurement unit 381 interrogates the WNR measurement sample to obtain optical signals corresponding to leukocytes. The analysis unit 851 of the control unit 200 analyzes the measurement results of the WNR measurement sample to obtain the leukocyte count.

Chamber C24 is a chamber for preparing a sample for reticulocyte measurement and a sample for optical measurement of platelets by the optical measurement unit 381. Diluent solution B housed in the first container 701 is supplied to the chamber C24 via the reserve tank RT2 (see FIG. 8). Furthermore, staining solution C is supplied to the chamber C24 from the third container 902, and staining solution D is supplied from the third container 903. Diluent solution B, staining solution C, and the sample are mixed to prepare a RET measurement sample, in which blood sample is diluted and reticulocytes are stained, used for reticulocyte measurement. The RET measurement sample prepared in the chamber C24 is measured by the optical measurement unit 381. The optical measurement unit 381 interrogates the RET measurement sample to obtain optical signals corresponding to reticulocytes. The analysis unit 851 of the control unit 200 analyzes the measurement results of the RET measurement sample and performs classification of reticulocytes.

In the chamber C24, diluent solution B, staining solution D, and the sample are mixed to prepare a PLT-F measurement sample, in which blood sample is diluted and platelets are stained, used for optical measurement of platelets. The PLT-F measurement sample prepared in the chamber C24 is measured by the optical measurement unit 381. The optical measurement unit 381 interrogates the PLT-F measurement sample to obtain optical signals corresponding to platelets. The analysis unit 851 of the control unit 200 analyzes the measurement results of the PLT-F measurement sample to obtain the platelet count.

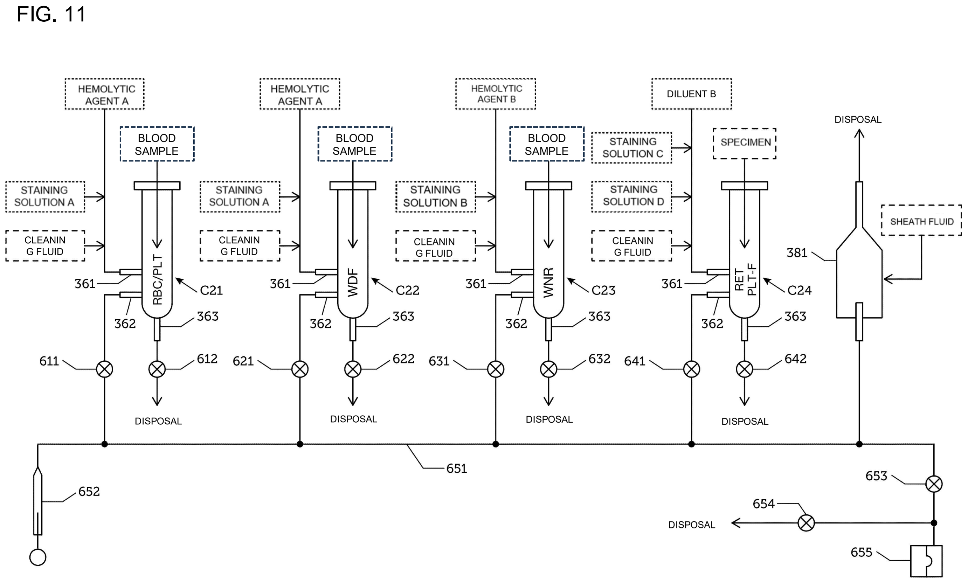

FIG. 11 is a diagram schematically showing an example of the configuration of a fluid circuit including the chambers C21 to C24 and the optical measurement unit 381.

Chambers C21 to C24 have similar configurations to each other. Each of the chambers C21 to C24 includes an inlet 361 to which reagents and cleaning fluid are supplied, an outlet 362 through which the measurement sample prepared in the chamber is discharged, and a disposal port 363 through which liquid in the chamber is discharged.

Hemolytic agent A, staining solution A, and cleaning fluid are supplied to the chamber C21 via the inlet 361. Hemolytic agent A, staining solution A, and cleaning fluid, similar to chamber C21, are supplied to the chamber C22 via the inlet 361. Hemolytic agent B, staining solution B, and cleaning fluid are supplied to the chamber C23 via the inlet 361. Diluent solution B, staining solution C, staining solution D, and cleaning fluid are supplied to the chamber C24.

The outlets 362 of the chambers C21 to C24 are respectively connected to a flow path 651 via valves 611, 621, 631, and 641. A syringe pump 652, valves 653, 654, and the optical measurement unit 381 are connected to the flow path 651. A diaphragm pump 655 is connected to the flow path 651 via the valve 653. The disposal ports 363 of the chambers C21 to C24 are respectively connected to a waste flow path via valves 612, 622, 632, and 642.

When the preparation of the measurement sample in each chamber shown in FIG. 11 is completed, the diaphragm pump 655 draws the prepared measurement sample from the corresponding chamber into the flow path 651. The syringe pump 652 supplies the measurement sample filled in the flow path 651 to the optical measurement unit 381. The syringe pump 652 is configured to be able to deliver a predetermined amount of the measurement sample within the flow path 651 to the optical measurement unit 381 by applying a predetermined pressure to the flow path 651.

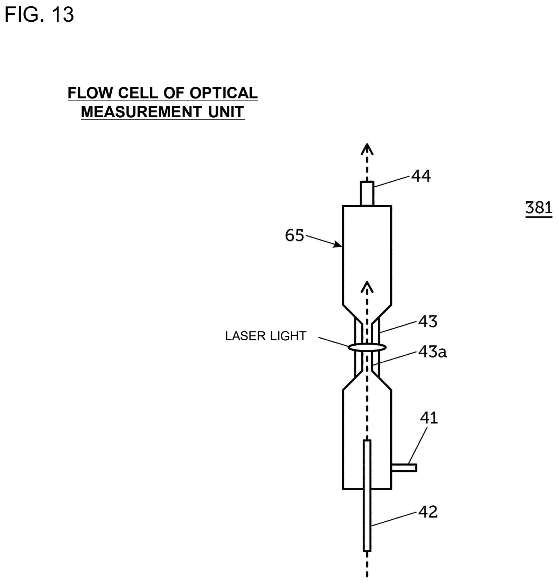

The optical measurement unit 381 causes the measurement sample supplied from the flow path 651 and sheath fluid to flow into a flow cell 65 (see FIG. 13) and outputs optical signals corresponding to blood cells in the measurement sample based on the flow cytometry method. The measurement sample that has passed through the optical measurement unit 381 is discarded. When the measurement by the optical measurement unit 381 for one measurement sample is completed, cleaning fluid is supplied to the chamber where the measurement sample was prepared. The cleaning fluid in the chamber is discharged to the flow path 651 via the outlet 362 and discarded via the disposal port 363. The cleaning fluid discharged into the flow path 651 is discarded via the valve 654 by the syringe pump 652 and the diaphragm pump 655.

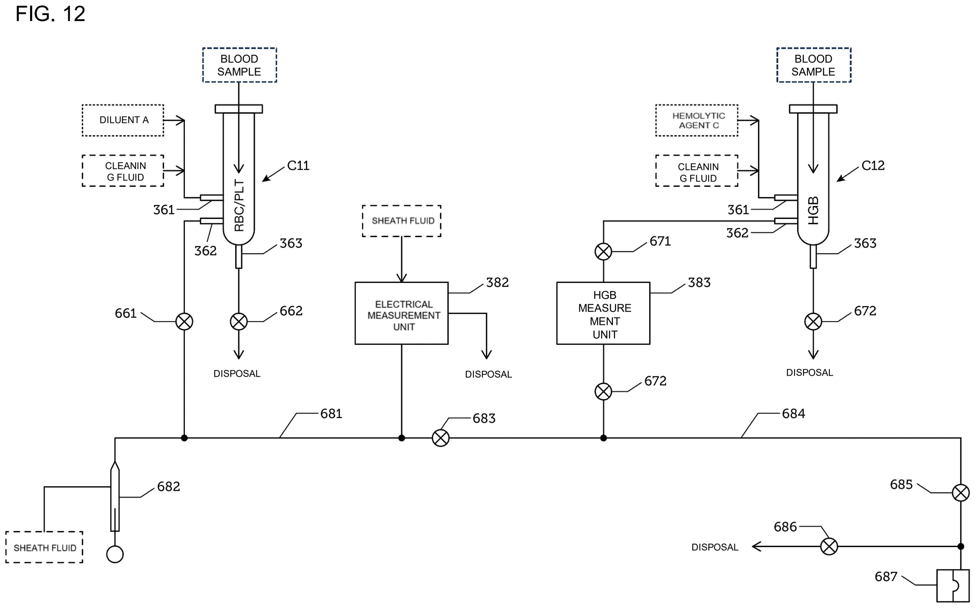

FIG. 12 is a diagram schematically showing an example of the configuration of a fluid circuit connected to the chamber C11, the HGB chamber C12, the electrical measurement unit 382, and the HGB measurement unit 383.

The chambers C11 and C12 have a configuration similar to the chambers C21 to C24 described above. Each of the chambers C11 and C12 includes an inlet 361 to which reagents and cleaning fluid are supplied, an outlet 362 through which the measurement sample prepared in the chamber is discharged, and a disposal port 363 through which liquid in the chamber is discharged.

Diluent solution A and cleaning fluid are supplied to the chamber C11 via the inlet 361. Hemolytic agent C is supplied to the HGB chamber C12 via the inlet 361.

The outlet 362 of the chamber C11 is connected to a flow path 681 via a valve 661. A syringe pump 682, a valve 683, and the electrical measurement unit 382 are connected to the flow path 681. The outlet 362 of the chamber C12 is connected to the HGB measurement unit 383 via a valve 671. The HGB measurement unit 383 is connected to a flow path 684 via a valve 672. Valves 683, 685, and 686 are connected to the flow path 684. A diaphragm pump 687 is connected to the flow path 684 via the valve 685. The disposal ports 363 of the chambers C11 and C12 are respectively connected to a waste flow path via valves 662 and 672.

When the preparation of the RBC/PLT measurement sample in the chamber C11 is completed, the diaphragm pump 687 draws the RBC/PLT measurement sample from the chamber C11 into the flow path 681. The syringe pump 682 supplies the RBC/PLT measurement sample stored in the flow path 681 to the electrical measurement unit 382. The syringe pump 682 is configured to be able to transfer a predetermined amount of the measurement sample stored in the flow path 681 to the electrical measurement unit 382 by applying a predetermined pressure to the flow path 681.

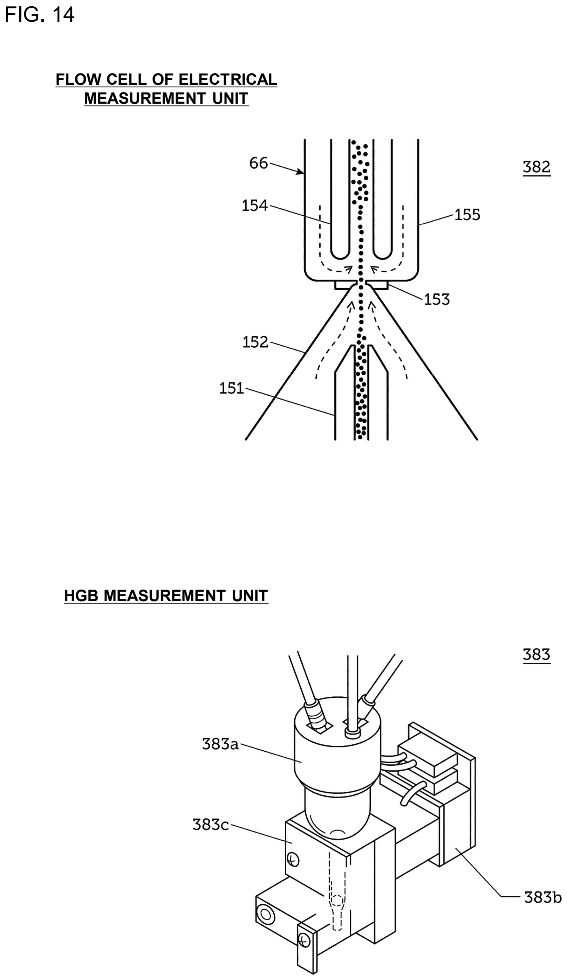

The electrical measurement unit 382 causes the RBC/PLT measurement sample supplied from the flow path 681 and sheath fluid to flow into a flow cell 66 (see FIG. 14) and outputs electrical signals corresponding to blood cells in the RBC/PLT measurement sample based on the sheath flow DC detection method. The RBC/PLT measurement sample that has passed through the electrical measurement unit 382 is discarded. When the measurement by the electrical measurement unit 382 for the RBC/PLT measurement sample is completed, cleaning fluid is supplied to the chamber C11. The cleaning fluid in the chamber C11 is discharged to the flow path 681 via the outlet 362 and discarded via the disposal port 363. The cleaning fluid discharged into the flow path 681 is discarded via the valve 686 by the syringe pump 682 and the diaphragm pump 687.

When the preparation of the HGB measurement sample in the chamber C12 is completed, the HGB measurement sample is supplied to the HGB measurement unit 383 via the valve 671. The HGB measurement unit 383 outputs the absorbance corresponding to the hemoglobin concentration. The HGB measurement sample used for measurement in the HGB measurement unit 383 is discarded. When the measurement by the HGB measurement unit 383 for the HGB measurement sample is completed, cleaning fluid is supplied to the chamber C12. The cleaning fluid in the chamber C12 is discharged to the HGB measurement unit 383 via the outlet 362 and discarded via the disposal port 363. The cleaning fluid discharged to the HGB measurement unit 383 is discharged to the flow path 684 through the interior of the HGB measurement unit 383. The cleaning fluid discharged into the flow path 684 is discarded via the valve 686 by the syringe pump 682 and the diaphragm pump 687.

FIG. 13 is a diagram showing the configuration of the flow cell 65 of the optical measurement unit 381.

As shown in FIG. 13, the flow cell 65 of the optical measurement unit 381 includes a sheath fluid supply port 41, a sample nozzle 42, a micro-aperture section 43, and a disposal port 44.

The sheath fluid supply port 41 supplies sheath fluid into the flow cell 65. The sample nozzle 42 injects the measurement sample upwards within the flow cell 65. The measurement sample, surrounded by sheath fluid, advances to the disposal port 44 through a flow path 43a formed in the micro-aperture section 43. Blood cells contained in the measurement sample pass through the flow path 43a one by one. The flow path 43a is irradiated with a laser beam of a predetermined wavelength. When the measurement sample passing through the flow path 43a is irradiated with the laser beam, forward scattered light, side scattered light, and fluorescence are generated from the blood cells in the measurement sample. The light receiving unit of the optical measurement unit 381 outputs optical signals corresponding to the intensity of the forward scattered light, side scattered light, and fluorescence. The intensity of the forward scattered light reflects information regarding the size of the blood cells, the intensity of the side scattered light reflects internal information of the blood cells, and the intensity of the fluorescence reflects the degree of staining of the blood cells. During cleaning of the optical measurement unit 381, cleaning fluid is supplied to the sheath fluid supply port 41 and the sample nozzle 42.

Note that the optical signal may be any signal obtained as an optical response by irradiating blood cells with light. The optical signal is not limited to signals based on light scattering and fluorescence as described above, and may be, for example, a signal based on light absorption or a signal based on transmitted light.

FIG. 14 is a diagram showing the configuration of the flow cell 66 of the electrical measurement unit 382 and the configuration of the HGB measurement unit 383.

As shown on the upper side of FIG. 14, the flow cell 66 of the electrical measurement unit 382 includes a sample nozzle 151, a chamber 152, an aperture 153, a collection tube 154, and a chamber 155.

The sample nozzle 151 sends the measurement sample upwards. The chamber 152 has a tapered shape that narrows toward the top. Sheath fluid is supplied into the chamber 152. The measurement sample, surrounded by sheath fluid, advances to the collection tube 154 through the aperture 153. Blood cells contained in the measurement sample pass through the aperture 153 one by one. Electrodes are provided around the aperture 153. A direct current is supplied between the electrodes of the aperture 153, and an electrical signal corresponding to the change in DC resistance when the measurement sample passes through the aperture 153 is detected. The RBC/PLT diluent solution used for the preparation of the RBC/PLT measurement sample is conductive because it contains an electrolyte. As a result, when blood cells in the RBC/PLT measurement sample pass through the aperture 153, the DC resistance increases, and thus the electrical signal reflects the information of the blood cells passing through the aperture 153.

Sheath fluid is supplied to the chamber 155 so as to flow downward in the outer region of the recovery tube 154. The sheath fluid flowing in outside path of the collection tube 154 flows into the inner path of the collection tube 154 after reaching the lower end of the chamber 155. This prevents blood cells that have passed through the aperture 153 from returning to the aperture 153 again, thereby preventing false detection of blood cells. During cleaning of the electrical measurement unit 382, cleaning fluid is supplied to the sample nozzle 151 and the chambers 152 and 155.

As shown on the lower side of FIG. 14, the HGB measurement unit 383 includes a cell 383a, a light source unit 383b, and a light receiving unit 383c.

The cell 383a is made of a highly transparent plastic material. The light source unit 383b irradiates the cell 383a with light of a wavelength that has high absorbance by SLS-hemoglobin. The light receiving unit 383c is arranged facing the light source unit 383b across the cell 383a and receives the transmitted light that has passed through the cell 383a.

The HGB measurement sample is housed in the cell 383a. In this state, the light source unit 383b emits light, and the light receiving unit 383c receives the transmitted light. Since the cell 383a is made of a highly transparent material, the light receiving unit 383c receives only the transmitted light from the light source unit 383b that was not absorbed by the HGB measurement sample. The light receiving unit 383c detects a signal corresponding to the intensity of the transmitted light. This signal corresponds to the absorbance.

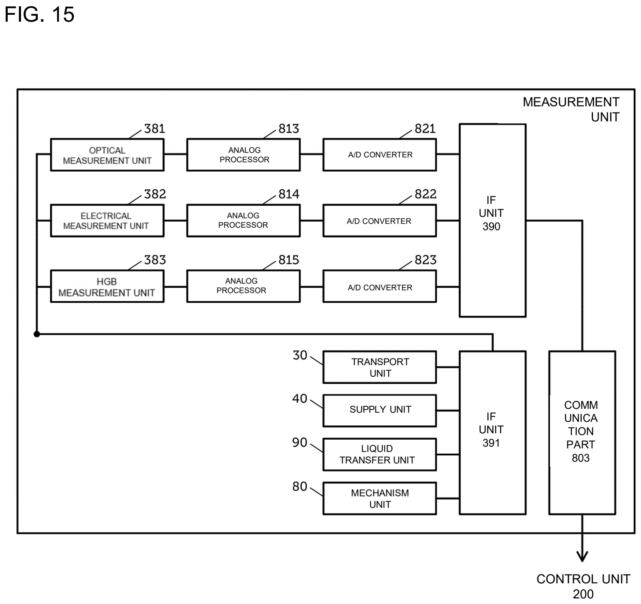

FIG. 15 is a block diagram showing an example of the configuration of the measurement unit 10.

The measurement unit 10 includes the optical measurement unit 381, the electrical measurement unit 382, the HGB measurement unit 383, analog processors 813, 814, 815, A/D converters 821, 822, 823, IF (interface) units 390, 391, a communication unit 803, a transport unit 30, a supply unit 40, and a liquid transfer unit 90.

The analog processors 813, 814, and 815 perform processing such as noise removal and smoothing on the analog signals output from the optical measurement unit 381, the electrical measurement unit 382, and the HGB measurement unit 383, respectively. The A/D converters 821, 822, and 823 convert the analog signals processed by the analog processing units 813, 814, and 815 into digital signals, respectively, and transmit them as measurement results to the control unit 200 via the IF unit 390 and the communication unit 803. The communication unit 803 is configured by a connection terminal based on the USB standard and communicates with the control unit 200.

The liquid transfer unit 90 includes a mechanism for driving the syringe pumps, diaphragm pumps, and valves for transferring liquid in the flow paths shown in FIGS. 11 and 12. The mechanism unit 80 includes a gripping mechanism 310 (see FIG. 3) for taking out the sample container 110 from the rack R, an agitation mechanism 320 (see FIG. 3) for agitating the sample container 110, and a dispensing mechanism 350 for moving the aspiration pipette 351 for aspirating the sample from the sample container 110 and dispensing it to each chamber. Each part of the measurement unit 10 is controlled by the control unit 200 via the IF unit 391 and the communication unit 803.

FIG. 16 is a block diagram showing an example of the configuration of the transport unit 30 and the control unit 200.

The transport unit 30 includes a mechanism unit 251 and a communication unit 252.

The mechanism unit 251 includes a mechanism for moving the rack R in the first region 31, a mechanism for moving the rack R in the second region 32, and a mechanism for driving the conveyor 33. The communication unit 252 is configured by a connection terminal based on the USB standard and communicates with the control unit 200.

The control unit 200 includes a control unit 850, a storage 861, a display unit 853, an input unit 854, and communication units 862, 863.

The control unit 850 is composed of, for example, a CPU. The control unit 850 functions as an analysis unit 851 for analyzing the sample and a measurement control unit 852 for controlling the measurement unit 10 by executing a computer program stored in the storage 861. The storage 861 is composed of, for example, an SSD, HDD, RAM, or the like. The storage unit 861 stores the measurement results received from the measurement unit 10, a program for controlling the control unit 200, and a program for realizing the functions of the analysis unit 851 and the measurement control unit 852. The storage unit 861 also stores a reagent information DB that stores reagent information for various reagents exclusively managed by the measuring apparatus 1.

The display unit 853 is composed of, for example, a liquid crystal display or an organic EL display. The input unit 854 is composed of a mouse, keyboard, or the like. Note that the display unit 853 and the input unit 854 may be integrally configured, for example, as a touch panel display.

The communication unit 862 is composed of a connection terminal based on the USB standard and communicates with the communication unit 803 of the measurement unit 10 and the communication unit 252 of the transport unit 30 via a cable based on the USB standard. The measurement control unit 852 controls each part of the transport unit 30 via the communication unit 862 and receives the sample ID read by the reading unit 34 of the transport unit 30.

The communication unit 863 is composed of a connection terminal based on the Ethernet standard and communicates with an external host computer of the measuring apparatus 1 via a cable based on the Ethernet standard. When the measurement control unit 852 receives the sample ID read by the reading unit 34 of the transport unit 30, the measurement control unit 852 queries the host computer for the measurement order corresponding to the sample ID via the communication unit 863. The measurement control unit 852 receives the measurement order corresponding to the sample ID from the host computer. The received measurement order for each sample is stored in the storage unit 861. The measurement control unit 852 can also accept a measurement order from an operator via the input unit 854.

The measuring apparatus 1 determines a measurement mode according to the measurement items designated in the measurement order. The measuring apparatus 1 is configured to be able to measure blood samples under, for example, the following eight measurement modes:

(1) CBC
(2)

$$CBC + DIFF$$

(3)

$$CBC + RET$$

(4)

$$CBC + DIFF + RET$$

(5)

$$CBC + PLT\text{-}F$$

(6)

$$CBC + DIFF + PLT\text{-}F$$

(7)

$$CBC + RET + PLT\text{-}F$$

(8)

$$CBC + DIFF + RET + PLT\text{-}F$$

The measurement items included in the measurement order are, for example, as follows:

- Measurement items corresponding to CBC (details described later, multiple measurement items including Red Blood Cell Count (RBC), etc.)
- Measurement items corresponding to DIFF (details described later, multiple measurement items including Neutrophil Count (NEUT#), Lymphocyte Count (LYMPH#), etc.)
- Measurement items corresponding to RET (details described later, measurement items including Reticulocyte Count, etc.)
- Measurement items corresponding to PLT-F (details described later, measurement items including Platelet Count, etc.)

CBC is an abbreviation for Complete Blood Count. The measurement items corresponding to CBC are the eight items: Red Blood Cell Count (RBC), White Blood Cell Count (WBC), Hemoglobin (HGB), Hematocrit (HCT), Mean Corpuscular Volume (MCV), Mean Corpuscular Hemoglobin (MCH), Mean Corpuscular Hemoglobin Concentration (MCHC), and Platelet Count (PLT). The measurement items corresponding to CBC may also include Nucleated Red Blood Cell Count (NRBC#), Nucleated Red Blood Cell Ratio (NRBC%), Basophil Count (BASO#), and Basophil Ratio (BASO%). Red Blood Cell Count (RBC), Platelet Count (PLT), and Hematocrit (HCT) are determined based on electrical signals obtained by the electrical measurement unit 382 measuring the RBC/PLT measurement sample. White Blood Cell Count (WBC) is determined based on optical signals obtained by the optical measurement unit 381 measuring the WNR measurement sample. Hemoglobin (HGB) is determined based on optical signals obtained by the HGB measurement unit 383 measuring the HGB measurement sample. Mean Corpuscular Volume (MCV) is determined from the Red Blood Cell Count (RBC) and Hematocrit (HCT). MCH (Mean Corpuscular Hemoglobin) is determined from the Red Blood Cell Count (RBC) and Hemoglobin (HGB). MCHC (Mean Corpuscular Hemoglobin Concentration) is determined from the Hematocrit (HCT) and Hemoglobin (HGB).

The measurement items corresponding to DIFF are, for example, Neutrophil Count (NEUT#), Lymphocyte Count (LYMPH#), Monocyte Count (MONO#), Eosinophil Count (EO#), Neutrophil Ratio (NEUT%), Lymphocyte Ratio (LYMPH%), Monocyte Ratio (MONO%), and Eosinophil Ratio (EO%). For the measurement items corresponding to DIFF, when the WDF measurement sample is measured by the optical measurement unit 381, leukocytes are classified into multiple subpopulations (neutrophils, lymphocytes, monocytes, eosinophils), and the classified subpopulations are counted, respectively. As described above, if CBC includes Basophil Count (BASO#) as a measurement parameter, leukocytes may be classified into five subpopulations (neutrophils, lymphocytes, monocytes, eosinophils, and basophils) based on the CBC measurement data and the DIFF measurement data, and the five classified subpopulations may be counted, respectively.

The measurement item corresponding to RET is, for example, Reticulocyte Count (RET#). The measurement items corresponding to RET may further include Reticulocyte Ratio (RET%), Low Fluorescence Reticulocyte Ratio (LFR), Medium Fluorescence Reticulocyte Ratio (MFR), and High Fluorescence Reticulocyte Ratio (HFR). For the measurement items corresponding to RET, when the RET measurement sample is measured by the optical measurement unit 381, mature red blood cells and reticulocytes are classified, and the classified reticulocytes are counted. The measurement items corresponding to RET may further include the Platelet Count (PLT#) obtained by optically measuring the platelet count as a parameter.

The measurement item corresponding to PLT-F is Platelet Count (PLT). The measurement items corresponding to PLT-F may further include Immature Platelet Fraction (IPF) and Immature Platelet Count (IPF#). For the measurement items corresponding to PLT-F, when the PLT-F measurement sample is measured by the optical measurement unit 381, platelets are classified, and the classified platelets are counted.

If the measurement items included in the measurement order are only those corresponding to CBC, the measurement control unit 852 determines CBC mode as the measurement mode. In the CBC mode, the measurement control unit 852 controls the measurement unit 10 to prepare the RBC/PLT measurement sample, the HGB measurement sample, and the WNR measurement sample.

If the measurement items included in the measurement order are those corresponding to CBC and DIFF, the measurement control unit 852 determines CBC + DIFF mode as the measurement mode. In the CBC + DIFF mode, the measurement control unit 852 controls the measurement unit 10 to prepare the RBC/PLT measurement sample, the HGB measurement sample, the WNR measurement sample, and the WDF measurement sample.

If the measurement items included in the measurement order are those corresponding to CBC and RET, the measurement control unit 852 determines CBC + RET mode as the measurement mode. In the CBC + RET mode, the measurement control unit 852 controls the measurement unit 10 to prepare the RBC/PLT measurement sample, the HGB measurement sample, the WNR measurement sample, and the RET measurement sample.

If the measurement items included in the measurement order are those corresponding to CBC, DIFF, and RET, the measurement control unit 852 determines CBC + DIFF + RET mode the measurement mode. In the CBC + DIFF + RET mode, the measurement control unit 852 controls the measurement unit 10 to prepare the RBC/PLT measurement sample, the HGB measurement sample, the WNR measurement sample, the WDF measurement sample, and the RET measurement sample.

If the measurement items included in the measurement order are those corresponding to CBC and PLT-F, the measurement control unit 852 determines CBC + PLT-F mode as the measurement mode. In the CBC + PLT-F mode, the measurement control unit 852 controls the measurement unit 10 to prepare the RBC/PLT measurement sample, the HGB measurement sample, the WNR measurement sample, and the PLT-F measurement sample.

If the measurement items included in the measurement order are those corresponding to CBC, DIFF, and PLT-F, the measurement control unit 852 determines CBC + DIFF + PLT-F mode as the measurement mode. In the CBC + DIFF + PLT-F mode, the measurement control unit 852 controls the measurement unit 10 to prepare the RBC/PLT, HGB, WNR, WDF, and PLT-F measurement samples.

If the measurement items included in the measurement order are those corresponding to CBC, RET, and PLT-F, the measurement control unit 852 determines CBC + RET + PLT-F mode as the measurement mode. In the CBC + RET + PLT-F mode, the measurement control unit 852 controls the measurement unit 10 to prepare the RBC/PLT measurement sample, the HGB measurement sample, the WNR measurement sample, the RET measurement sample, and the PLT-F measurement sample.

If the measurement items included in the measurement order are those corresponding to CBC, DIFF, RET, and PLT-F, the measurement control unit 852 determines CBC + DIFF + RET + PLT-F mode as the measurement mode. In the CBC + DIFF + RET + PLT-F mode, the measurement control unit 852 controls the measurement unit 10 to prepare the RBC/PLT, HGB, WNR, WDF, RET, and PLT-F measurement samples.

The measurement unit 10 supplies the measurement samples prepared in each measurement mode to the optical measurement unit 381, the electrical measurement unit 382, and the HGB measurement unit 383, interrogates respective measurement samples, and transmits the measurement data obtained from each measurement sample to the control unit 200. The control unit 850 of the control unit 200 stores the acquired measurement data in the storage unit 861.

The analysis unit 851 analyzes the measurement data of the RBC/PLT measurement sample and obtains the red blood cell count, platelet count, and mean corpuscular volume (MCV), etc., as analysis results. The mean corpuscular volume is a measured value related to the volume of red blood cells. The analysis unit 851 analyzes the measurement data of the HGB measurement sample and obtains the hemoglobin concentration, etc., as analysis results. The analysis unit 851 classifies blood cells in the sample into neutrophils, lymphocytes, monocytes, eosinophils, etc., based on the measurement data of the WDF measurement sample, and analyzes the measurement data of the WNR measurement sample to classify blood cells in the sample into basophils, etc. Based on the classification results of the WDF measurement sample and the WNR measurement sample, the analysis unit 851 classifies blood cells in the sample into neutrophils, lymphocytes, monocytes, eosinophils, and basophils, and obtains the number and/or ratio of neutrophils, lymphocytes, monocytes, eosinophils, and basophils as analysis results. The analysis unit 851 analyzes the measurement data of the WNR measurement sample to classify blood cells in the sample into white blood cells, nucleated red blood cells, etc., and obtains the white blood cell count and nucleated red blood cell count as analysis results. The analysis unit 851 analyzes the measurement data of the RET measurement sample to classify blood cells in the sample into reticulocytes, etc., and obtains the reticulocyte count, etc., as analysis results. The analysis unit 851 analyzes the measurement data of the PLT-F measurement sample to classify blood cells in the sample into platelets, etc., and obtains the platelet count, etc., as classification results.

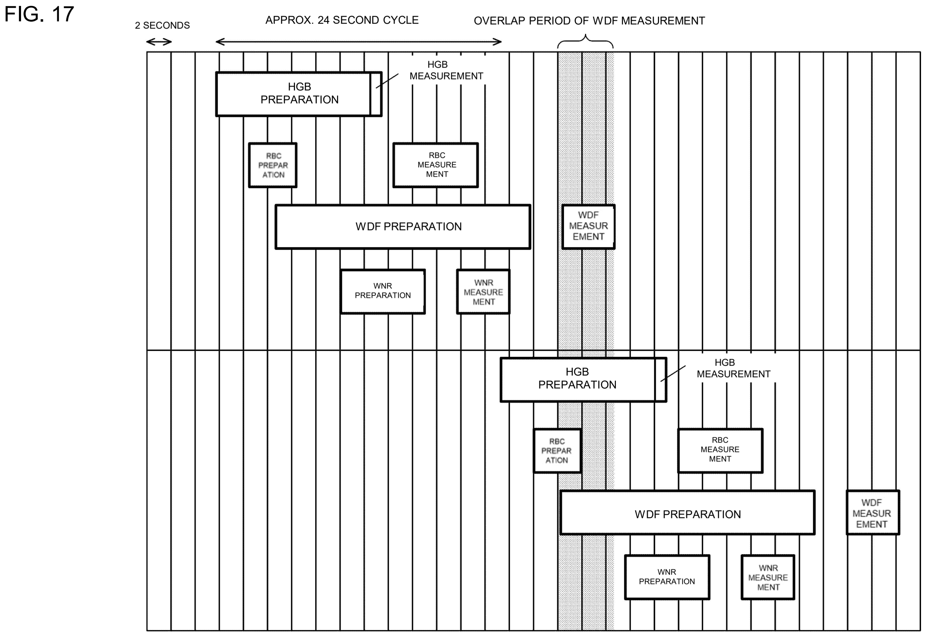

FIG. 17 is a time chart of measurement performed by the first measurement unit 11. The first measurement unit 11 is configured to run measurement sequences for two blood samples in a partially overlapping manner, enabling a throughput of, for example, 150 blood samples/hour in the CBC + DIFF mode. The second measurement unit 12 is configured similarly to the first measurement unit 11. In this configuration, the measurement unit 10 is capable of measuring at a throughput of 300 blood samples/hour in the CBC + DIFF mode. The following describes the time chart for two consecutively measured blood samples with reference to FIG. 17.

In the time chart shown in FIG. 17, the right direction indicates the passage of time, and the time width between two adjacent vertical lines is 2 seconds. The measurement control unit 852 controls the preparation of the measurement sample in each chamber and the measurement by each measurement unit, as shown in the time chart below. The measurement control unit 852 executes one operation sequence for one sample. The operation sequence defines the order and timing for operating each part of the measurement unit 10, namely the sample preparation unit (i.e., chambers C11, C12, C21 to C24), the respective measurement units 381 to 383, and components such as valves and pumps included in the fluid circuit. When the measurement control unit 852 executes the operation sequence, the valves are controlled to open at a predetermined timing, for example, and the valves and pumps are respectively controlled to drive the pumps after the predetermined time. As will be described in detail below, the measurement control unit 852 starts the operation sequence at a predetermined timing, enabling the preparation of the WDF measurement sample and the measurement by the optical measurement unit for two consecutive samples in a partially overlapping manner.

In FIG. 17, the rectangular labels indicate the duration of the steps included in the operation sequence. The left end of the rectangular label for preparation indicates the timing when the mixing of reagents into the chamber starts, and the right end indicates the timing when the transfer of the reacted measurement sample from the chamber starts. The left end of the rectangular label for measurement indicates the start timing of the signal acquisition step in the corresponding detection unit, and the right end indicates the end timing of the signal acquisition step.

As shown in FIG. 17, when the CBC + DIFF mode is set corresponding to the measurement order, the steps of "HGB preparation" for preparing the HGB sample, "RBC preparation" for preparing the RBC/PLT sample, "WDF preparation" for preparing the WDF sample, and "WDF preparation" (likely an error, should be "WNR preparation") for preparing the WNR sample are executed in sequence. These steps are executed as follows. First, the sample aspirated by the suction tube 301 is dispensed sequentially into chambers C12, C11, C21, and C22. The predetermined reagents mentioned above, namely diluent solution, hemolytic agent, and staining solution, are supplied to each chamber by the liquid transfer unit 90 for preparing each sample. The sample and the reagents are mixed in each chamber, and the measurement sample is prepared after a predetermined reaction time has elapsed.

When the preparation of the measurement sample in each chamber is completed, the measurement sample is transferred from each chamber to the corresponding measurement unit, and measurement is executed in each measurement unit. The transfer of the measurement sample and the measurement by each measurement unit are performed, for example, as follows.

When the HGB preparation step is completed, the valve 671 in the flow path between the chamber C12 and the HGB measurement unit 383 is opened, and a predetermined amount of the HGB measurement sample is transferred to the HGB measurement unit 383. In the "HGB measurement" step, the transferred HGB sample is measured by the HGB measurement unit 383.

When the RBC preparation step is completed, the diaphragm pump 687 draws the RBC/PLT measurement sample from the chamber C11, and the syringe pump 682 transfers the RBC measurement sample to the electrical measurement unit 382. In the "RBC measurement" step, the transferred RBC/PLT sample is measured by the electrical measurement unit 382.

When the WDF preparation step is completed, the diaphragm pump 655 draws the WDF measurement sample from the chamber C21, and the syringe pump 652 transfers the WDF measurement sample to the optical measurement unit 381. In the "WDF measurement" step, the transferred WDF sample is measured by the optical measurement unit 381.

When the WNR preparation step is completed, the diaphragm pump 655 draws the WNR measurement sample from the chamber C23, and the syringe pump 652 transfers the WNR measurement sample to the optical measurement unit 381. In the "WNR measurement" step, the transferred WNR sample is measured by the optical measurement unit 381.

In this way, the CBC + DIFF measurement of the first sample is performed. If the second sample following the first sample is also measured in the CBC + DIFF mode, HGB preparation/measurement, RBC preparation/measurement, WDF preparation/measurement, and WNR preparation/measurement are executed for the second sample as well, similar to the first sample. The preparation time and measurement time for each sample are the same for the first and second samples, but the chamber used for WDF preparation is different. If the WDF sample for the first sample was prepared in chamber C21, the WDF sample for the subsequent second sample is prepared in chamber C22. In other words, when measurements are continuously performed in the CBC + DIFF mode, the two chambers C21 and C22 are used alternately.

When WDF measurements of multiple samples are executed continuously, the period from WDF preparation to WDF measurement for the first and second samples is executed with at least a partial overlap, as indicated by hatching in FIG. 17. In the example of FIG. 16, the WDF preparation of the second sample is executed overlapping with the WDF measurement of the first sample. More specifically, the WDF preparation of the second sample is started between the WDF preparation and WDF measurement of the first sample. According to such a configuration, the WDF preparation of the second sample can be started without waiting for the completion of the WDF measurement of the first sample, thus significantly improving the throughput of the measurement unit 10.

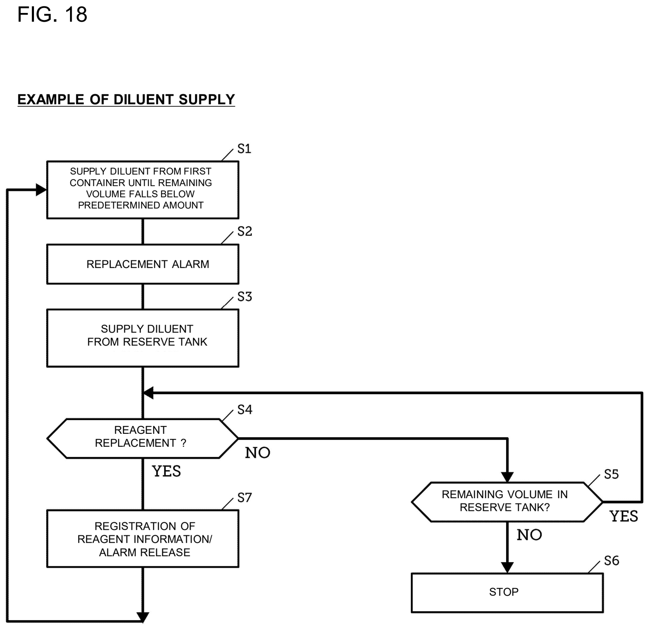

FIG. 18 is a flowchart concerning the supply of diluent solution by the measuring apparatus 1. The continuous loading function of the measuring apparatus 1 using the reserve tank RT will be described with reference to FIG. 18. Although FIG. 18 describes the flow for supplying the diluent solution from the first container 700 as an example, the supply of hemolytic agent from the second container 800 is also executed by the same flow. Furthermore, the following description explains an example of supplying diluent solution A via the reserve tank RT1, but other reagents (diluent solution B, hemolytic agents A to C) are also supplied via the corresponding reserve tanks RT2 to RT5, respectively.

<Step S1: Supply of Diluent>

In step S1, the measurement control unit 852 controls the supply unit 40 to continue the supply of the diluent solution from the first container 700 to the measurement unit 10 until the remaining amount of the diluent solution in the first container 700 falls below a predetermined amount. The supply of the diluent solution to the measurement unit 10 includes, for example, supplying diluent solution A from the first container 700 accommodating diluent solution A to the chamber C11 for the preparation of the RBC sample in the chamber C11 mentioned above. If diluent solution A is used as the cleaning fluid for the cleaning of the chambers C11, C12, and C21 to C24, the supply of the diluent solution in step S1 may include supplying diluent solution A as the cleaning fluid to respective chambers.

The control of the supply unit 40 by the measurement control unit 852 is performed, for example, as follows.

(1) Filling the Reserve Tank

The measurement control unit 852 drives the pump 57 connected to the first container 700 accommodating diluent solution A to store a predetermined amount of diluent solution A in the reserve tank RT1. For example, the reserve tank RT1 is equipped with a float sensor FS as described with reference to FIG. 7, and the measurement control unit 852 is configured to detect the liquid level of diluent solution A based on the signal from the float sensor FS. The measurement control unit 852 fills the reserve tank RT1 with diluent solution A until the liquid level reaches a predetermined upper limit position, based on the float sensor FS.

(2) Supply to the Measurement Unit 10

The measurement control unit 852 controls the supply unit 40 to supply diluent solution A from the reserve tank RT1 to the measurement unit 10 according to the measurement operation by the measurement unit 10. For example, referring to the time chart shown in FIG. 17, in the "RBC preparation" step, a predetermined amount of diluent solution A stored in the reserve tank RT1 is supplied to the chamber C11, and the sample aspirated from the sample container 110 is mixed with the predetermined amount of diluent solution A. The supply unit 40 includes a metering unit that includes valves and pumps for metering and drawing diluent solution A from the reserve tank RT1 and transferring the liquid to the chamber connected to the reserve tank RT1. The measurement control unit 852 controls the metering unit of the supply unit 40 to perform the metering of diluent solution A and the liquid transfer to the chamber C11.

(3) Replenishment of the Reserve Tank

As the diluent solution is supplied from the reserve tank RT1 to the measurement unit 10, the remaining amount of diluent solution A in the reserve tank RT1 decreases, and the liquid level of diluent solution A consequently drops. When the measurement control unit 852 detects, based on the signal from the float sensor FS, that the remaining amount of diluent solution A in the reserve tank RT1 has decreased to a predetermined replenishment level (e.g., 80% of the maximum level), the measurement control unit 852 drives the pump 57 again to supply diluent solution A from the first container 700 to the reserve tank RT1 until the water level of diluent solution A reaches the predetermined upper limit position. In this way, the diluent solution A is replenished to the reserve tank RT1 as needed so that a constant amount, for example, an amount between the maximum level (100%) and the replenishment level (80%), is stored. Note that although (2) and (3) are described here as being executed sequentially, (2) and (3) may be executed simultaneously. That is, if the pump 57 is driven while diluent solution A is being drawn from the bottom of the reserve tank RT1 according to the measurement operation by the measurement unit 10, diluent solution A is replenished from the first container 700 to the reserve tank RT1 while diluent solution A is being supplied from the reserve tank RT1.

(4) Diluent Remaining Volume Detection When the replenishment from the first container 700 to the reserve tank RT1 is repeated in response to the supply from the reserve tank RT1 to the measurement unit 10, the diluent solution A in the first container 700 decreases, and eventually the remaining amount of diluent solution A in the first container 700 falls below a predetermined amount. The predetermined amount is, for example, an amount that cannot be further aspirated by the tube inserted into the container, i.e., the dead volume remaining in the container. The supply unit 40 includes a sensor for detecting the remaining amount in the first container 700 housing diluent solution A. In the example of FIG. 7, the supply unit 40 includes a bubble sensor BS in the flow path connecting the reserve tank RT1 and the first container 700. The bubble sensor BS detects air bubbles entering into the flow path when the diluent solution A is exhausted, at the time the diluent solution A is aspirated via the first tube 51a inserted into the first container 700. The measurement control unit 852 detects that the remaining amount in the first container 700 has fallen below the predetermined amount, based on the signal from the bubble sensor BS. When the measurement control unit 852 detects that the remaining amount is below the predetermined amount, the process proceeds to step S2.

In the above example, a bubble sensor is used to detect the remaining quantity of the reagent in the first container 700, but the remaining amount may be detected by other methods. For example, a float sensor may be provided on the first tube 51a inserted into the first container 700 to detect the remaining amount based on the liquid level in the first container 700. Alternatively, the remaining amount may be detected based on the number of tests performed using the diluent in the first container 700. For example, the remaining amount may be determined by subtracting the number of tests already performed from the initial number of tests available for a new (unused) container.

<Step S2: Output Replacement Alarm>

In step S2, the measurement control unit 852 outputs a replacement alarm. The replacement alarm may be any content that urges the user of the measuring apparatus 1 to replace the first container 700, and various output formats can be adopted. The output formats can be, for example, (1) screen display, (2) voice message, or (3) notification to a mobile terminal. Examples of each output format are described below.

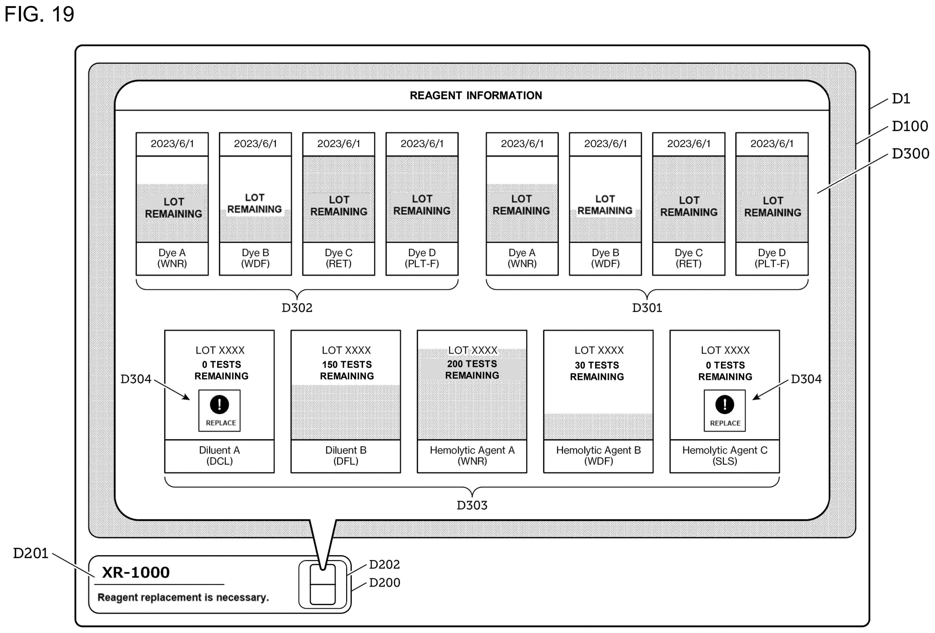

An example of the screen display of the replacement alarm will be described with reference to FIG. 19.

The replacement alarm is displayed on a display by the control unit 200, for example. The control unit 200 manages the remaining reagent volume in cooperation with the supply unit 40. The control unit 200 manages the remaining reagent volume based on the one-to-one relationship between the supply unit 40 and the corresponding measurement unit 10. The control unit 200 manages the remaining reagent volume concerning the supply unit 40 as the remaining volume of the reagent used exclusively for the corresponding measurement unit 10. The control unit 200 manages the remaining reagent volume of each container corresponding to the supply unit 40 based on the amount of reagent exclusively supplied to the measurement unit 10 corresponding to the supply unit 40, in the screen illustrated in FIG. 19. FIG. 19 shows an example of a screen D1 displayed on the display 70. The screen D1 includes a first area D100 and a second area D200 as main display areas. The first area D100 displays, for example, icons for the user to give instructions to the control unit 200, and analysis results of samples generated by the control unit 200. The second area D200 displays the status of the measurement unit 10. For example, if an error occurs in the measurement unit 10, a notification that an error has occurred is displayed in the second area D200. In FIG. 19, as an example of the output of the replacement alarm, a message D201 indicating that reagent replacement is necessary is displayed as an example of an error that has occurred in the measurement unit 10. The user can recognize that reagent replacement is necessary for the measurement unit 10 by checking the display in the second area D200. In FIG. 19, the replacement alarm is displayed in the form of a message, but it may be output in other forms. For example, an icon representing the status of the measurement unit 10 by color may be displayed, and a change in the color of the icon may indicate that the reagent has run out. For example, the icon may be displayed in green when the measurement unit 10 is normal, and in red when the reagent has run out.

When the user selects the second area D200 with a pointing device on the screen of FIG. 19, a reagent information window D300 is displayed. In the screen example of FIG. 19, the reagent information window D300 is displayed overlaid on the first display area 100, but the information in the reagent information window D300 may be displayed in the first display area 100. The reagent information window D300 includes an area D301 for displaying information about the reagents housed in the first measurement unit 11, an area D302 for displaying information about the reagents housed in the second measurement unit 20, and an area D303 for displaying information about the reagents housed in the reagent housing unit 50.

The area D301 displays multiple reagent volume gauges indicating the remaining volume of multiple types of staining solutions set in the reagent placement unit 23 of the first measurement unit 11. For example, in the example of FIG. 19, four reagent volume gauges corresponding to staining solutions A, B, C, and D are displayed from the left. The graphic of each reagent volume gauge includes a rectangular frame and a gauge representing the remaining volume of the staining solution inside the frame. The gauge displays the remaining volume of each staining solution by height. For example, the gauge represents the height of the remaining volume of the staining solution when the amount of staining solution housed in an unused third container is set to 100%. By displaying the remaining volume of the staining solution graphically in this way, the user can visually grasp the remaining volume of each reagent. Furthermore, by displaying the remaining volume of multiple staining solutions housed in the first measurement unit 11 aggregated on a single screen, the user can view the remaining volume of the multiple reagent staining solutions in a single view.

The area D302 displays multiple reagent volume gauges indicating the remaining volume of multiple types of staining solutions set in the reagent placement unit 23 of the second measurement unit 20. The configuration of the area D302 is the same as the area D301.

The area D303 displays multiple reagent volume gauges indicating the remaining volume of multiple types of reagents housed in the reagent housing unit 50. In the example of FIG. 19, five reagent volume gauges corresponding to diluent solution A, diluent solution B, hemolytic agent A, hemolytic agent B, and hemolytic agent C are displayed from the left. In the example of FIG. 19, the measurable number of tests for diluent solution A and hemolytic agent C are zero, and the gauges corresponding to these reagents are represented by a height of zero.

As illustrated in FIG. 19, the remaining volume of each reagent may be displayed by text in addition to the reagent volume gauge. The remaining volume may be displayed as, for example, "250/500," indicating that the current remaining volume is 250 tests out of a maximum (unused) volume of 500 tests, or it may be displayed in the form of a measurable number of tests, such as "100 tests remaining." By checking the measurable number of tests, the user can grasp how many measurements are possible before the reagent runs out. The measurable number of tests is an example of the display format, and the remaining volume of the reagent may be displayed by volume, such as "50 ml remaining," or the remaining volume may be displayed qualitatively in multiple stages (e.g., 5 stages). As shown in FIG. 19, the reagent's Lot Number (LOT) and Expiration Date (EXP) may be displayed in addition to the remaining volume information for the various reagents.

An icon D304 indicating that reagent replacement is necessary is additionally displayed on the reagent volume gauges for diluent solution A and hemolytic agent C. The user can recognize the type of reagent that has run out by checking the reagent volume gauge. Furthermore, by adding and displaying the icon D304 on the gauge, the reagents that have run out (diluent solution A and hemolytic agent C in the example of FIG. 19) are emphasized and distinguished from other reagents. The emphasis facilitates the user's recognition of the type of reagent that needs to be replaced. The emphasis is not limited to the display of the icon D304. For example, the gauge for a reagent that has run out and needs replacement may be displayed in a first color (e.g., red) for warning, and the color of the gauges for other reagents that have not run out may be displayed in a second color (e.g., blue). These are examples of outputting the replacement alarm by screen display.

The output of the replacement alarm by voice message is performed, for example, by the measurement control unit 852 outputting a voice message such as "Please replace the reagent container for Diluent Solution A" from a speaker (not shown), in response to detecting that diluent solution A in the first container 700 has run out.

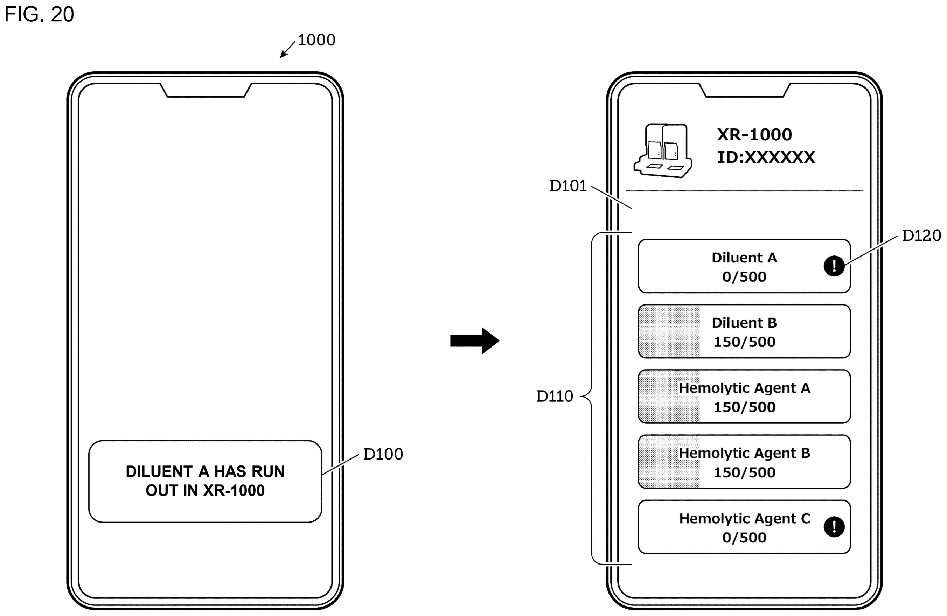

FIG. 20 is an example of the output of an replacement alarm by notification to a mobile terminal. The mobile terminal 1000 is a portable electronic terminal such as a smartphone, tablet computer, or laptop computer. In the example of FIG. 20, the mobile terminal 1000 is a smartphone. The mobile terminal 1000 is connected to the internet via a mobile communication network such as 5G, for example. An application program that assists the operation of the measuring apparatus 1 is installed on the mobile terminal 1000, for example. The mobile terminal 1000 can communicate with a cloud server that collects and provides data from the measuring apparatus 1. The communication unit 863 of the control unit 200 is also connected to the internet and can communicate with the aforementioned cloud server. When the measurement control unit 852 of the measuring apparatus 1 detects that diluent solution A has run out, the measurement control unit 852 sends data indicating that diluent solution A has run out to the cloud server, associated with the device ID of the measuring apparatus 1. Upon receiving this data, the cloud server generates a message indicating that diluent solution A has run out and sends a push notification to the mobile terminal 1000. When the application on the mobile terminal 1000 receives the push notification, the mobile terminal 1000 displays a message indicating that diluent solution A has run out on the display unit. FIG. 20 shows an example where the mobile terminal 1000, which received the push notification, displays a message indicating that diluent solution A has run out. In this way, by displaying the replacement alarm on the mobile terminal 1000 additionally to or alternatively to the display on the display 70 of the measuring apparatus 1, even if the user is away from the measuring apparatus 1, the user can grasp that the reagent has run out and promptly start the reagent replacement. Furthermore, by displaying the message via a push notification, the user can recognize the replacement alarm without opening the application.

The mobile terminal 1000 may display the reagent remaining volume information of the measurement unit 10 according to the user's operation of the application. The right side of FIG. 20 is an example of an application screen. The application screen D101 includes, for example, the ID of the data-linked measurement unit 10 and the reagent information of the measurement unit 10. In the example of FIG. 20, the remaining volumes of the multiple reagents set in the measuring apparatus 1 are displayed by reagent volume gauges, similar to the reagent information displayed on the reagent information screen shown in FIG. 18. In the example of FIG. 20, only the remaining volumes of a part of the multiple reagents set in the measuring apparatus 1 are displayed according to the screen size of the mobile terminal 1000. The user can display the remaining volumes of other reagents by scrolling the screen. In the example of FIG. 20, five reagent volume gauges corresponding to diluent solution A, diluent solution B, hemolytic agent A, hemolytic agent B, and hemolytic agent C are displayed from top to bottom. The layout of the screen example in FIG. 20 is different from the screen in FIG. 18: the reagent volume gauges are arranged vertically to suit the smartphone screen size, with the right end of each reagent volume gauge corresponding to 100% remaining volume and the left end corresponding to 0%.

Similarly to FIG. 18, the screen example in FIG. 20 shows a state where diluent A and hemolytic agent C have run out. An icon D120, indicating that reagent replacement is necessary, is added to the reagent volume gauges for diluent A and hemolytic agent C. This allows the user to identify the reagents that need replacement via the icon D120. By not only sending a notification to the mobile terminal 1000 but also allowing the user to check the remaining reagent volumes of the measuring apparatus 1 from the mobile terminal 1000, a user who receives an alarm notification does not need to return to the apparatus solely to check the remaining volumes, even from a remote location. When replacement is required, it is preferable for the user to avoid multiple trips between the measuring apparatus 1 and the inventory space by bringing not only the urgently needed reagents but also other reagents that are running low. Therefore, enabling the remaining volumes of all reagents to be checked via the application streamlines the reagent replacement workflow.

<Step S3: Supply from Reserve Tank> Referring again to FIG. 18 , in step S3, the measurement control unit 852 stops the supply of diluent solution A from the first container 700 and supplies the diluent solution from the reserve tank RT1 to the measurement unit 10. In step S3, similar to step S1, the diluent solution A stored in the reserve tank RT1 is supplied to the measurement unit 10 according to the consumption of diluent solution A in the measurement unit 10. At the time of step S3, the amount of diluent solution in the first container 700 is less than or equal to the predetermined amount, and the remaining amount of diluent solution A in the reserve tank RT1 is at the replenishment level (e.g., 80%). The measurement control unit 852 controls the supply unit 40 to continue supplying the diluent solution A stored in the reserve tank RT1 to the measurement unit 10 without further aspirating diluent solution A from the first container 700.

The supply unit 40 continues the supply of diluent solution A even while the user is performing the replacement work for the first container 700 in response to the replacement alarm. The replacement of the first container 700 is performed, for example, as follows. The user opens the opening/closing part 61 (see FIG. 4) to expose the reagent housing unit 50 and pulls the drawer 52 on which the first container 700 is placed forward. The second tube 51b extending from the reserve tank RT1 has sufficient length to prevent it from becoming disconnected from the first container 700 when the drawer 52 is pulled out. The user takes the empty first container 700 that has run out of reagent out of the drawer and places a new first container 700 on the drawer 52. The user removes the connector 53 from the empty first container 700, pulls out the first tube 51a, inserts the first tube 51a into the new first container 700, and attaches the connector 53. The user pushes the drawer 52 with the new first container 700 placed on it back in and closes the opening/closing part 61. In this way, the container that has run out of reagent is replaced with a new container.

The supply unit 40 continues the supply of the diluent solution from the reserve tank RT even when the opening/closing part 61 is opened for the replacement of the first container 700 housing the diluent solution. Similarly for the hemolytic agent, the supply unit 40 continues the supply of the hemolytic agent from the reserve tank RT even when the opening/closing part 61 is opened for the replacement of the second container 800 housing the hemolytic agent.

Furthermore, the supply unit 40 continues the supply of the diluent solution from the reserve tank RT even when the first tube 51a is pulled out from the first container 700 housing the diluent solution. Similarly for the hemolytic agent, the supply unit 40 continues the supply of the hemolytic agent from the reserve tank RT even when the opening/closing part 61 is opened for the replacement of the second container 800 housing the hemolytic agent.

<Step S4: Determine Reagent Exchange> In step S4, the measurement control unit 852 determines whether the exhausted reagent container has been replaced. This determination of placement may be made based on, for example, (1) receipt of new reagent information (e.g., user input), or (2) detection of an automatic replacement via a signal from a sensor.

As an example of the above (1), the measurement control unit 852 may recognize reagent replacement upon the input of reagent information of the same type as the reagent that is the subject of the replacement alarm. For example, when a replacement alarm for Diluent A has been output, the measurement control unit 852 may determine that the reagent has been replaced when reagent information for a new first container 700 storing Diluent A is input by the user. The reagent information can be input to the control unit 200 by having an unillustrated reading device, such as a handheld code reader, read the machine-readable code printed on the outside of the first container 700. The machine-readable code includes reagent information such as the reagent type code (a code to distinguish between Diluent A, Diluent B, and Hemolytic Agents A to C), the expiration date, and the lot number of the reagent stored in the container. The reagent information is input to the measurement control unit 852 by decoding the code read by the code reader. Alternatively, the user may input the reagent information to the control unit 200 by operating a keyboard or touch panel. If the type code included in the input reagent information matches the type of reagent for which the replacement alarm is output, the measurement control unit 852 determines YES in step S4 and advances the process to step S7.

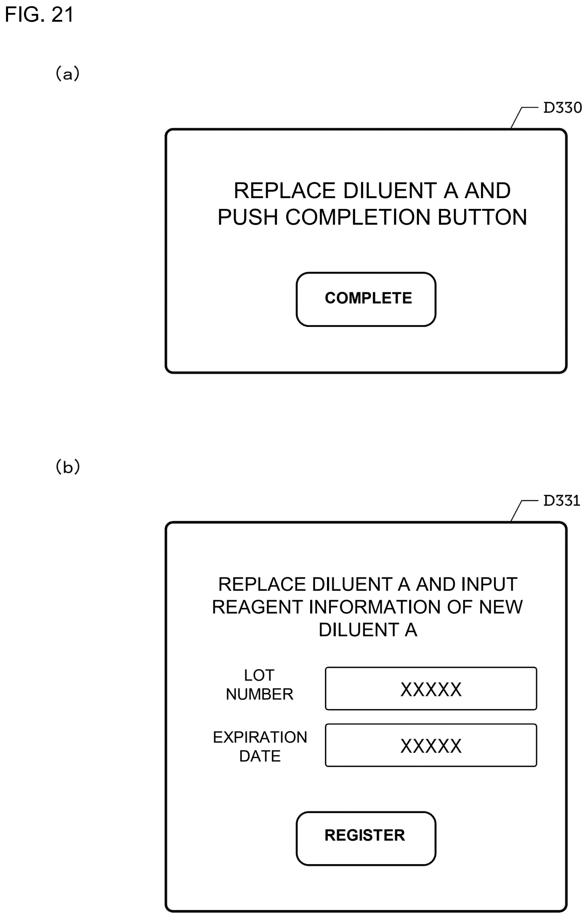

As another example of the above (1), the measurement control unit 852 may recognize that the reagent has been replaced by accepting an operation to release the replacement alarm. For example, when the replacement alarm D304 corresponding to Diluent A is selected in the screen example of FIG. 19, the measurement control unit 852 displays a dialog box D330 illustrated in FIG. 21A. The message "Please replace Diluent A and select the Completion button" is displayed in the dialog box D330. When the user replaces Diluent A and selects the Completion button, the measurement control unit 852 recognizes that the reagent has been replaced and advances the process to step S5. Alternatively, when the replacement alarm D304 corresponding to Diluent A is selected, the measurement control unit 852 may display a dialog box D331 illustrated in FIG. 21B. The message "Please replace Diluent A and input the reagent information for the new Diluent A" is displayed in the dialog box D331. The dialog box D331 further includes an input field for the lot number and an input field for the expiration date of the new Diluent A. When the user replaces Diluent A, inputs the information in each field, and selects the "Register" button, the measurement control unit 852 recognizes that the reagent has been replaced and advances the process to step S7.

As an example of the above (2), the measurement control unit 852 may recognize that the reagent has been replaced, for example, by aspirating the reagent from the new first container 700 and detecting no air bubbles during aspiration.

<Step S5: Reserve Tank Remaining Volume Check> If it is determined in step S4 that the reagent has not been replaced, the measurement control unit 852 determines whether the remaining volume of Diluent A in the reserve tank RT1 is equal to or greater than a predetermined volume. Whether the remaining volume is equal to or greater than the predetermined volume is determined, for example, by whether the float sensor FS provided in the reserve tank RT1 has reached the lower limit level. If the float sensor FS has not reached the lower limit level, a predetermined volume or more of Diluent A remains in the reserve tank RT1, so the measurement control unit 852 continues to supply Diluent A to the measurement unit 10 and returns the process to the determination in step S4.

<Step S6: Stopping Measurement Unit 10> If it is determined in step S5 that the remaining volume in the reserve tank RT1 is not equal to or greater than the predetermined volume, the measurement control unit 852 stops the measurement of new blood samples by the measurement unit 10. Stopping the measurement of new blood samples includes, for example, suspending the aspiration of the blood sample that the aspiration tube 351 of the measurement unit 10 is next scheduled to aspirate. In step S6, while the measurement of new blood samples is stopped, the processing of blood samples that have already been aspirated by the aspiration tube 351 and for which measurement has started is continued. By continuing the measurement of aspirated blood samples, waste of aspirated blood samples can be avoided. Here, the "predetermined volume" mentioned in step S5 is set to be more than the amount required to complete the measurement of at least the already aspirated blood samples. Furthermore, the "predetermined volume" is set to correspond to the maximum number of blood samples that the measurement unit 10 can measure simultaneously. For example, as described with reference to FIG. 17, the first measurement unit 11 of this embodiment is configured to be able to measure two blood samples simultaneously to enhance throughput. For example, the second measurement unit 12 is similarly configured. In this example configuration, the measurement unit 10 is configured to be able to measure a maximum of four blood samples simultaneously. Therefore, the "predetermined volume" in this example configuration is set to be more than the amount required to complete the measurement of at least four blood samples. This makes it possible to continue the throughput of the measurement unit 10 for as long as possible without wasting the aspirated blood samples.

<Step S7: Reagent Information Registration/Alarm Release> If it is determined in step S4 that the reagent replacement has been performed, the measurement control unit 852 updates the reagent information DB stored in the storage unit 861 based on the reagent information of the replaced reagent. For example, when Diluent A is replaced, the remaining volume information, expiration date, and lot number of Diluent A stored in the reagent information DB are each updated based on the information of the new first container 700. The remaining volume information may be overwritten based on the input reagent information if remaining volume information is stored in the input reagent information, or it may be reset to the initial remaining volume value in response to the reagent replacement if remaining volume information is not included in the input reagent information; the expiration date and lot number are overwritten with the newly input information, respectively. After registering the post-replacement reagent information, the measurement control unit 852 releases the replacement alarm output in step S2. When the measurement control unit 852 releases the alarm, it returns the process to step S1.

Although FIG. 18 describes the flow of supplying Diluent from the first container 700 as an example, the supply of Hemolytic Agent from the second container 800 is also executed by the same flow. Describing the Hemolytic Agent supply flow based on FIG. 18, in step S1, the supply unit 40 continues the supply of Hemolytic Agent from the second container 800 to the measurement unit 10 via the reserve tanks RT3 to 5 until the Hemolytic Agent in the second container 800 falls below a predetermined remaining volume. (1) Filling the reserve tank with Hemolytic Agent, (2) supplying Hemolytic Agent to the measurement unit 10, (3) replenishing the reserve tank with Hemolytic Agent, and (4) detecting the remaining volume of Hemolytic Agent are as described in the example of Diluent supply.

When replenishment from the second container 800 is repeated in response to the supply of Hemolytic Agent from the reserve tanks RT3 to 5 to the measurement unit 10, the Hemolytic Agent in the second container 800 decreases, and eventually the remaining volume falls below a predetermined volume. When the remaining volume of Hemolytic Agent in the second container 800 falls below the predetermined volume, the measurement control unit 852 outputs a replacement alarm in step S2. In step S3, the supply unit 40 stops the supply of Hemolytic Agent from the second container 800 and continues the supply of Hemolytic Agent from the reserve tanks RT3 to 5 to the measurement unit 10.

The supply unit 40 continues the supply of Hemolytic Agent even while the user is performing the replacement of the second container 800 upon receiving the replacement alarm. The replacement of the second container 800 is performed in the same manner as the replacement of the first container.

The supply unit 40 continues the supply of Hemolytic Agent from the reserve tank RT even when the opening/closing unit 61 is opened for the replacement of the second container 800 storing the Hemolytic Agent.

Furthermore, the supply unit 40 continues the supply of Hemolytic Agent from the reserve tank RT even when the first tube 51a is pulled out from the second container 800 storing the Hemolytic Agent.

In step S4, the measurement control unit 852 determines whether the exhausted reagent container has been replaced. When determining that the reagent container has not been replaced, the measurement control unit 852 determines in step S5 whether the remaining volume of Hemolytic Agent in the reserve tank RT is equal to or greater than a predetermined volume. If a predetermined volume of Hemolytic Agent remains, the process returns to the determination in step S4.

If it is determined in step S5 that the remaining volume in the reserve tank RT1 is not equal to or greater than the predetermined volume, the measurement control unit 852 stops the measurement of new blood samples by the measurement unit 10.

If it is determined in step S4 that the reagent replacement has been performed, the measurement control unit 852 updates the reagent information DB stored in the storage unit 861 based on the reagent information of the replaced reagent.

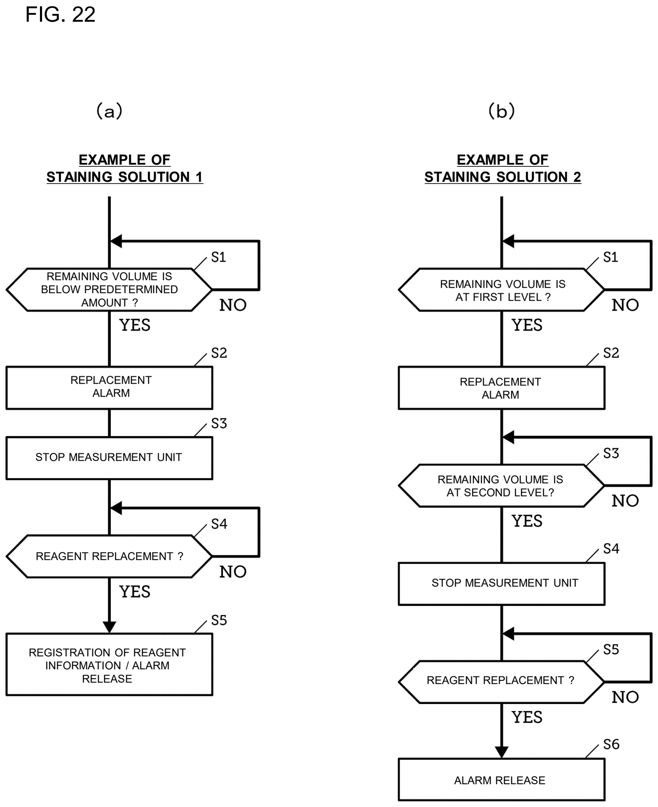

FIG. 22A is a flowchart related to the supply of staining solution by the measurement apparatus 1. Hereinafter, the supply of Staining Solution A among Staining Solutions A to D will be described as a representative example. In step S1, the measurement control unit 852 supplies the staining solution from the third container 900 to the measurement unit 10 until the remaining volume of the staining solution in the third container 900 falls below a predetermined volume. The determination in step S1 may be made based on, for example, whether the usable count of the staining solution stored in the storage unit 861 has reached a predetermined value (e.g., zero).

In step S2, when the remaining volume of the staining solution in the third container 900 falls below the predetermined volume, the measurement control unit 852 outputs a replacement alarm for the staining solution. The method for outputting the replacement alarm is as described with reference to FIG. 19.

In step S3, the measurement control unit 852 stops the measurement of new blood samples by the measurement unit 10. In step S3, while the measurement of new blood samples is stopped, the processing of blood samples that have already been aspirated by the aspiration tube 351 and for which measurement has started is continued.

In step S4, the measurement control unit 852 determines whether the third container storing the staining solution has been replaced. The determination of whether the third container has been replaced may be performed based on, for example, as in the case of the first container 700, the registration of the reagent information for the third container, or the aspiration of the staining solution from the newly set third container without air bubble detection.

In step S5, the measurement control unit 852 registers the reagent information and releases the replacement alarm. The registration of the reagent information is the same as described in step S7 of FIG. 18. In the supply of staining solution, unlike the example of diluent supply, the reserve tank RT1 is not used. When the staining solution in the third container runs out, the measurement of new blood samples in the measurement unit 10 is stopped.

FIG. 22B is a flowchart showing an example of staining solution supply according to a modified example. In FIG. 22A, the replacement alarm is output and the measurement of new blood samples by the measurement unit 10 is stopped in response to the remaining volume falling below a predetermined volume. In other words, the replacement alarm is not output until the measurement of new blood samples is stopped. In contrast, in FIG. 22B, the remaining amount is monitored stepwise at a plurality of levels; a replacement alarm is output when the remaining amount falls below a first level, and the measurement of new blood samples is stopped when the remaining amount falls below a second level. This will be described in detail below.

In step S1, the measurement control unit 852 determines whether the remaining volume of the staining solution in the third container is equal to or less than a first level. The remaining volume of the first level is greater than the second level, which will be described later. For example, when the amount of staining solution in an unused third container is 100%, the amount of staining solution at the first level is 10%, and the amount of staining solution at the second level is 0%. When the amount of staining solution in the third container reaches the first level, the measurement control unit 852 outputs a replacement alarm in step S2. The method for outputting the replacement alarm is as described with reference to FIG. 19. Even after outputting the replacement alarm, the measurement control unit 852 continues the supply of staining solution from the third container to continue the measurement of new blood samples.

In step S3, the measurement control unit 852 determines whether the amount of staining solution in the third container has reached the second level. In step S4, if the remaining volume reaches the second level, the measurement control unit 852 stops the measurement of new blood samples. In steps S5 and S6, after the measurement of new blood samples is stopped, when the third container is replaced, the measurement control unit 852 releases the replacement alarm.

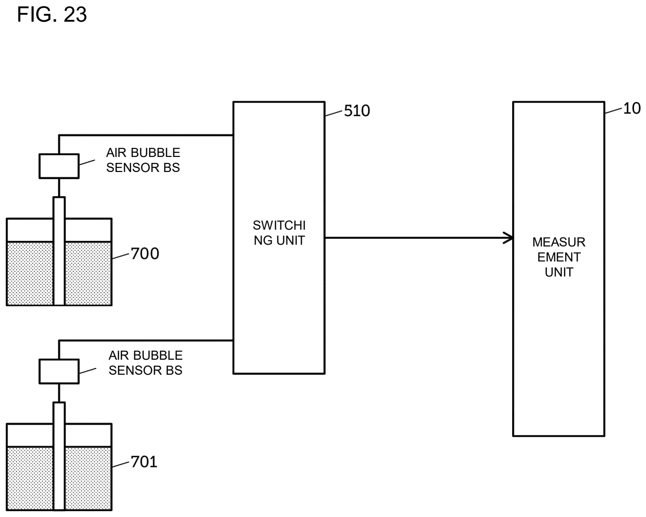

<Embodiment 2> The second embodiment will be described with reference to FIGS. 23 to 26. While the first embodiment described above achieves the continuous supply function for the diluent and hemolytic agent using the reserve tank RT, the second embodiment achieves the same function by implementing an automatic switching mechanism for the reagent supply source. Specifically, the measurement apparatus 1 of the second embodiment is configured to connect a plurality of containers storing the same type of reagent to the measurement unit, and automatically switches the supply source to a backup container when the container currently in use runs out of reagent. This enables the measurement apparatus 1 to continue supplying the reagent to the measurement unit even while the exhausted reagent container is being replaced.

FIG. 23 is a diagram showing an example configuration of the supply unit 40 according to Embodiment 2. For simplification of description, FIG. 23 only shows the fluid circuit for supplying Diluent A to the measurement unit 10 within the supply unit 40. The reagent storage unit 50 of Embodiment 2 includes a first container 700 and a fourth container 702 as containers for storing Diluent A. The first container 700 is fluidically connected to a switching unit 510 via an air bubble sensor BS. The fourth container 702 is fluidically connected to the switching unit 510 via an air bubble sensor BS. As described with reference to FIG. 5, the first tube 51a extending from the switching unit 510 is inserted into the opening of the container, fluidically connecting each container 700, 701 to the switching unit 510.

The switching unit 510 is a fluid mechanism for switching the supply source of Diluent A to be supplied to the measurement unit 10 between the first container 700 and the fourth container 702, and includes a valve for switching between, for example, a first flow path connecting the first container 700 and the measurement unit 10, and a second flow path connecting the fourth container 702 and the measurement unit 10. The switching unit 510 switches between the first flow path and the second flow path by driving the valve under the control of the measurement control unit 852.

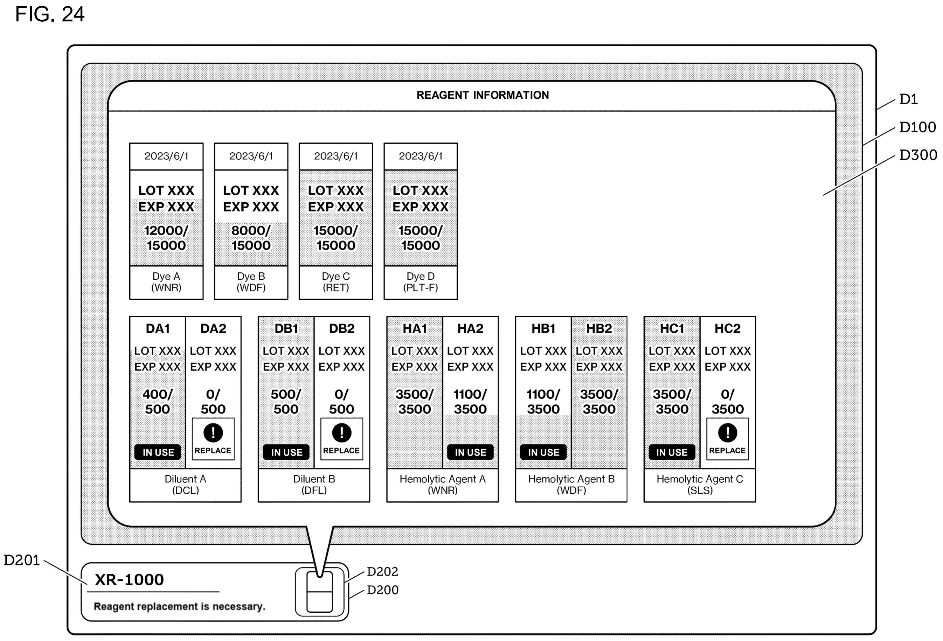

FIG. 24 is an example of the reagent information screen in Embodiment 2. Unlike the screen of Embodiment 1 illustrated in FIG. 19, the reagent volume gauges for Diluent A, Diluent B, Hemolytic Agent A, Hemolytic Agent B, and Hemolytic Agent C are displayed as twin displays, each including two gauges. Of the two gauges, one gauge corresponds to one reagent container, and the other gauge corresponds to the other reagent container. For example, in the Diluent A reagent volume gauge, the left gauge corresponds to the first container 700, and the right gauge corresponds to the fourth container 702. In the example of FIG. 24, of the two gauges for Diluent A, the left gauge is labeled with "DA1", representing "Diluent A_1," and the right gauge is labeled with "DA2", representing "Diluent A_2," and the two are displayed distinctly.

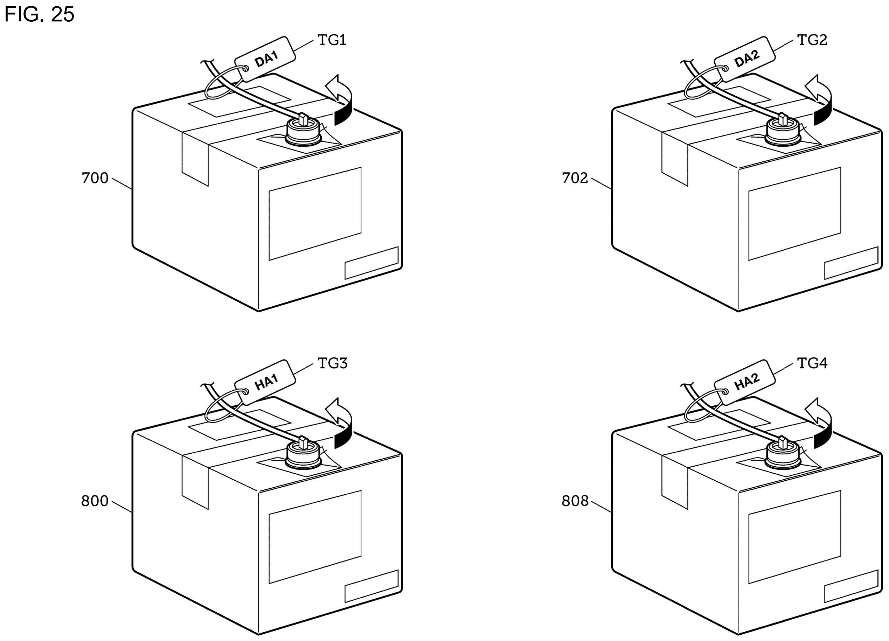

FIG. 25 is a diagram showing an example of identification information attached to the containers stored in the reagent storage unit 50. As shown in FIG. 25, a tag TG1 printed with identification information indicating that the tube corresponds to DA1 is attached to the tube inserted into the first container 700, and a tag TG2 printed with identification information indicating that the tube corresponds to DA2 is attached to the tube inserted into the fourth container 702. Since the remaining reagent volume inside the first and fourth containers is not visible, it is impossible to distinguish which is the first container and which is the fourth container based solely on appearance, and the out-of-reagent container can only be identified, for example, by lifting the box and checking its weight. By attaching tags as shown in FIG. 25, the user can identify the first container 700 and the fourth container 702 solely by sight, which can reduce the effort required for reagent replacement.

Furthermore, as shown in FIG. 25, the tags are printed with information to identify the type of reagent. In the example of FIG. 25, since the text printed on the tag is "DA", it is known that the reagent type is Diluent A. Similarly, for Diluent B, the tags are printed with "DB1" and "DB2." For Hemolytic Agent A, as shown in FIG. 25, a tag TG3 printed with "HA1", representing "Hemolytic Agent A_1," is attached to the second container 800 storing Hemolytic Agent A. A tag TG4 printed with "HA2", representing "Hemolytic Agent A_2," is attached to the fifth container 808 storing Hemolytic Agent A. Since the text printed on the tag is "HA", the user can know that the reagent type is Hemolytic Agent A. The user can recognize the type of reagent from the information printed on the tag. This prevents the user from accidentally using the wrong reagent. For example, it can prevent unintended problems such as a user viewing the reagent information screen of FIG. 24 and mistakenly pulling the tube from the second container 800 corresponding to the currently used "HA1," even though the user is supposed to replace the fourth container 702 corresponding to "DA2." Alternatively, if two tubes are removed from containers to replace two types of reagents at once, it becomes less likely to insert the tubes into the wrong containers.

FIG. 25 showed an example of attaching a tag printed with text to a tube as an example of identifying the reagent type. The method for identifying the reagent type is not limited to this; for example, the color of the tube may differ for each reagent type or for each type of container into which it should be inserted. For example, the tubes inserted into the Diluent A containers may be blue, the tubes inserted into the Diluent B containers may be green, and the tubes inserted into the Hemolytic Agent A containers may be purple, or other color coding may be used. Alternatively, the shape of the connector 53 (see FIG. 5) for attaching the tube to the container may differ for each type of container. For example, the connector 53 of the tube for DA1 may be designed to only engage with the screw of the Diluent A container, and the connector 53 of the tube for HA1 may be designed to only engage with the screw of the Hemolytic Agent A container.

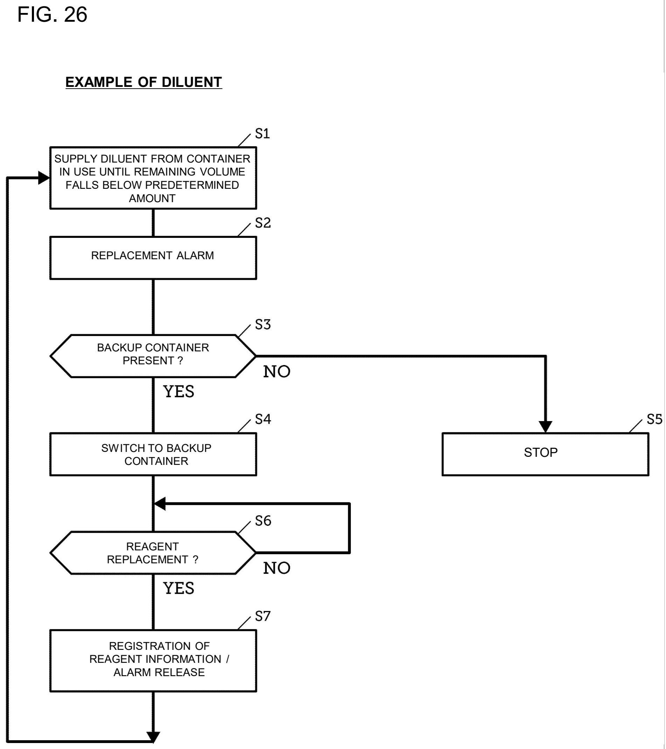

FIG. 26 is a flowchart related to the automatic switching of reagents. FIG. 26 illustrates the case of supplying Diluent A from the first container 700 and the fourth container 702 to the measurement unit 10 as an example. Since the automatic switching function is the same for Diluent B and Hemolytic Agents A to C, the description is omitted. The following description assumes a state where the first container 700 and the fourth container 702 are filled with the maximum amount of Diluent A, and the first container 700 is set to "In Use".

In step S1, the measurement control unit 852 controls the switching unit 510 to connect the flow path for supplying Diluent A from the first container 700 to the measurement unit 10. In this state, the measurement control unit 852 controls the supply unit 40 to supply Diluent A from the first container 700 to the measurement unit 10 until the remaining volume of Diluent A in the first container 700 falls below a predetermined volume. The determination of whether the volume has fallen below the predetermined volume is as described in step S1 of FIG. 18. When Diluent A is supplied from the first container 700 and the remaining volume of Diluent A falls below the predetermined volume, the air bubble sensor BS provided corresponding to the first container 700 detects air bubbles.

In step S2, the measurement control unit 852 outputs a replacement alarm corresponding to the first container 700. In the example of the reagent information screen in FIG. 24, the "Replace" icon is added to the DA1 reagent volume gauge and displayed. This notifies the user that DA1 has run out of reagent and requires replacement, prompting the replacement. The method for outputting the replacement alarm is as described with reference to FIG. 18 in Embodiment 1.

As shown in FIG. 24, on the reagent information screen, the "In Use" icon is added to the gauge corresponding to the reagent container currently in use among the two gauges. In the example of FIG. 24, the "In Use" icon is added to the DA1 gauge corresponding to the first container 700. Similarly, for other reagent types, the "In Use" icon is added to the gauge corresponding to the container currently in use among the two gauges. The user can ascertain which container is currently in use by checking the "In Use" icon.

The "Replace" icon is added to the gauge corresponding to the container that has run out of reagent, prompting the user to replace the reagent. In the example of FIG. 24, the "Replace" icon is added to the DA2 gauge corresponding to the fourth container 702. Similarly, for other reagent types, the "In Use" icon is added to the gauge corresponding to the container that has run out of reagent among the two gauges. The user prepares the container corresponding to the reagent with the "Replace" icon, and replaces the exhausted first container 700 with a new container by reconnecting the tube.

After the measurement control unit 852 outputs the replacement alarm, in step S3, the measurement control unit 852 determines whether a backup container corresponding to the out-of-reagent container is connected to the measurement unit 10. For example, when the first container 700 runs out of reagent, the measurement control unit 852 determines whether the fourth container 702 is connected as a backup container. The determination of whether a backup container is connected is made based on, for example, the remaining volume of the other container storing the same type of reagent as the container set to "In Use." For example, when the first container 700 (DA1) storing Diluent A is in use, if the remaining volume of the fourth container 702 (DA2) storing Diluent A is greater than zero, the measurement control unit 852 determines that a backup container is present. If the first container 700 (DA1) is in use and the remaining volume of the fourth container 702 (DA2) is zero, the measurement control unit 852 determines that no backup container is present. If the measurement control unit 852 determines that no backup container is present, the measurement control unit 852 stops the measurement of new blood samples in step S5.

If it is determined in step S3 that a backup container is present, in step S4, the measurement control unit 852 switches the supply source of Diluent A from the first container 700 (DA1) to the fourth container 702 (DA2). For example, the measurement control unit 852 controls the valve included in the switching unit 510 to close the first flow path connecting the first container 700 and the measurement unit 10, and open the second flow path connecting the fourth container 702 and the measurement unit 10. In this state, the measurement control unit 852 controls the supply unit 40 to supply Diluent A from the fourth container 702. The measurement control unit 852 changes the status of the fourth container 702 to "In Use" instead of the first container 700. The measurement control unit 852 also adds the "In Use" icon to the "DA2" reagent volume gauge on the reagent information screen.

In step S6, the measurement control unit 852 determines whether the exhausted reagent container has been replaced. Determination on whether the container has been replaced is as described in step S4 of FIG. 18.

In step S7, the measurement control unit 852 specifies the type of the replaced reagent based on the reagent type information included in the input reagent information. The measurement control unit 852 overwrites the reagent information of the container that is not in the "In Use" status among the two containers corresponding to the specified reagent type, with the newly input reagent information. For example, assume that the reagent information for the container storing Diluent A is input when the first container 700 (DA1) is out of reagent and the status of the fourth container 702 (DA2) is "In Use." In this case, the measurement control unit 852 automatically specifies the reagent information corresponding to the first container 700 (DA1) as the reagent information to be overwritten. The measurement control unit 852 overwrites the specified reagent information of the first container 700 (DA1) with the newly input reagent information. The reagent information of the fourth container 702 (DA2) is maintained without being overwritten. By maintaining the reagent information of the container currently in use and automatically overwriting the reagent information of the container not in use, the user does not need to input which of the two containers' reagent information should be overwritten, which reduces the complexity of reagent replacement. Furthermore, problems caused by a user mistakenly overwriting the reagent information of the container in use can be avoided. After registering the reagent information, the measurement control unit 852 releases the alarm and returns the process to step S1.

When the process returns to step S1, the container in use and the backup container swap roles from the previous turn. That is, in the previous turn, the status of the first container 700 (DA1) was "In Use" and the fourth container 702 (DA2) was the reserve, but in this turn, the first container 700 (DA1) is the reserve, and the fourth container 702 (DA2) is in use. Therefore, in this turn, in step S1, the supply of Diluent A continues until the remaining volume of the fourth container 702 falls below a predetermined volume. The same applies to the steps from S2 onward.

As described with reference to FIG. 26, in Embodiment 2, the measurement control unit 852 continues the supply of Diluent A until the remaining volume of the container currently in use falls below a predetermined volume, and then automatically switches the supply source of Diluent A to the backup container. According to this control, since the reagent in the other container is consumed only after the reagent in one of the two containers runs out, the reagent in the other container is preserved without the remaining volume being reduced until the first container runs out. Since the container currently in use is completely exhausted before switching to the other container, the other container still has a full or sufficient volume when replacement becomes necessary. This maximizes the time available for continuous loading and increases the likelihood of maintaining uninterrupted measurement without stopping the measurement unit 10. The significant effect of this can be understood when compared with a case where the reagents in the two containers are consumed simultaneously. Consider a case where the reagents in the two containers are consumed simultaneously, for example, by repeating a cycle of consuming a fixed amount of reagent from container A and then consuming a fixed amount of reagent from container B multiple times. In this case, on one hand, reagent replacement does not occur until the total amount of the reagents in containers A and B is exhausted, which extends the interval between reagent replacements. On the other hand, container A and container B will either run out simultaneously, or container B will have very little reagent remaining when container A runs out. As a result, it becomes impossible to secure sufficient time to replace the reagent without stopping the measurement unit 10. Therefore, Embodiment 2 described above is advantageous from the perspective of realizing continuous loading.

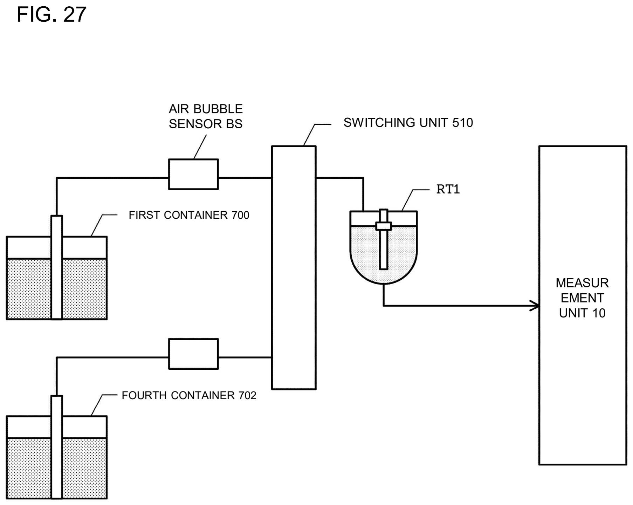

<Embodiment 3> The measurement apparatus of Embodiment 3 is a measurement apparatus equipped with the reserve tank feature of the measurement apparatus of Embodiment 1 and the switching feature of the measurement apparatus of Embodiment 2. FIG. 27 is a schematic diagram showing an example configuration of the supply unit 40 in Embodiment 3. The supply unit 40 of Embodiment 3 includes a reserve tank RT added between the switching unit 510 and the measurement unit 10 in the supply unit 40 of Embodiment 2.

The configuration of the reserve tank RT is the same as described in Embodiment 1. As described in Embodiment 2, the switching unit 510 includes a valve for switching the supply source of Diluent A between the first container 700 and the fourth container 702. The switching unit 510 can switch between a first flow path that fluidically connects the first container 700 and the reserve tank RT and a second flow path that fluidically connects the fourth container 702 and the reserve tank RT. Other configurations are the same as described in Embodiments 1 and 2.

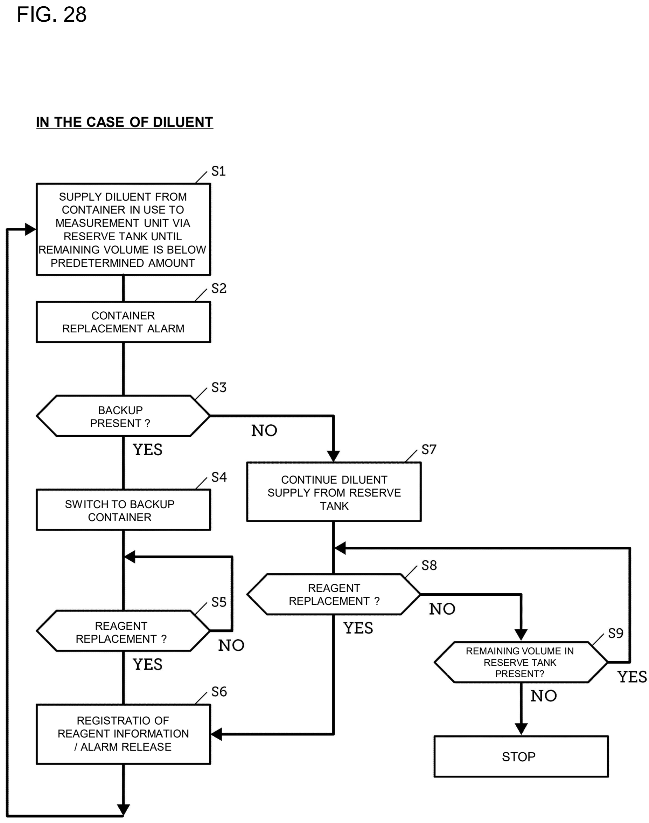

FIG. 28 is a flowchart showing an example of Diluent supply in Embodiment 3. FIG. 28 illustrates the case of supplying Diluent A from the first container 700 and the fourth container 702 to the measurement unit 10 via the reserve tank RT as an example. Since the function of supply via the reserve tank RT and automatic switching is the same for Diluent B and Hemolytic Agents A to C, the description is omitted. The following description assumes a state where the first container 700 and the fourth container 702 are filled with the maximum amount of Diluent A, and the first container 700 is set to "In Use".

In step S1, the measurement control unit 852 controls the supply unit 40 to supply Diluent A from the first container 700 currently in use to the measurement unit 10 via the reserve tank RT until the remaining volume of Diluent in the first container 700 falls below a predetermined volume.

In step S2, when the remaining volume of the first container 700 falls below the predetermined volume, the measurement control unit 852 causes a replacement alarm to be output. The method for outputting the replacement alarm is as described in Embodiments 1 and 2.

In step S3, the measurement control unit 852 determines whether a backup container is present. The determination of the presence or absence of a backup container is as described in Embodiment 2. If it is determined that a backup container is present, in step S4, the measurement control unit 852 switches the supply source of Diluent A from the first container 700 currently in use to the fourth container 702 as the backup container.

In step S5, the measurement control unit 852 determines whether the reagent has been replaced. The determination of the presence or absence of reagent replacement is as described in Embodiment 2. If it is determined that the reagent has been replaced, the measurement control unit 852 advances the process to step S6 and overwrites the reagent information corresponding to the out-of-reagent first container 700 with the newly input reagent information. The measurement control unit 852 releases the replacement alarm and returns the process to step S1.

If it is determined in step S3 that no backup container is present, the process advances to step S7. In step S7, the measurement control unit 852 stops the supply of Diluent from the container to the reserve tank RT and continues the supply of Diluent from the reserve tank RT.

In step S8, the measurement control unit 852 determines whether the reagent has been replaced. The determination of the presence or absence of reagent replacement is as described in Embodiment 2. In this case, there are two out-of-reagent containers, but which container's reagent to replace may be determined according to an arbitrary rule. For example, if both containers run out of reagent, the user may be prompted to preferentially replace the first container 700 (DA1), or the user may be allowed to select which of the first container 700 (DA1) and the fourth container 702 (DA2) to replace. If it is determined that the reagent has been replaced, the measurement control unit 852 advances the process to step S5 and overwrites the reagent information corresponding to the out-of-reagent container with the newly input reagent information.

If it is determined that the reagent has not been replaced, the measurement control unit 852 determines in step S9 whether there is a remaining volume in the reserve tank RT. The method for checking the remaining volume in the reserve tank RT is as described in Embodiment 1. If it is determined that there is a remaining volume in the reserve tank RT, the measurement control unit 852 returns the process to step S8 and repeats the determination until the reagent is replaced or the remaining volume in the reserve tank RT is exhausted. If it is determined in step S9 that the remaining volume in the reserve tank RT has been exhausted, the measurement control unit 852 stops the measurement of new blood samples.

According to Embodiment 3, by providing the reserve tank RT in addition to the plurality of containers storing the same type of reagent, it becomes possible to secure time for reagent replacement without stopping the measurement, thereby further enhancing the continuous loading function.

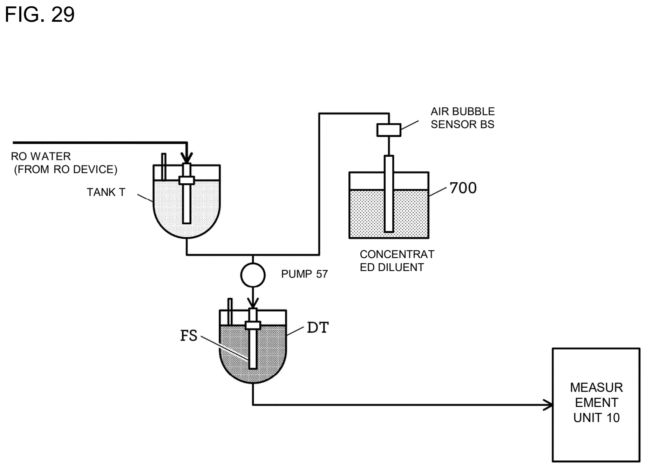

<Embodiment 4> Embodiment 4 will be described with reference to FIG. 29 . The measurement apparatus 1 of Embodiment 4 includes a tank T storing water, a first container 700 storing concentrated diluent, and a dilution tank DT. Embodiment 4 achieves the continuous loading function by diluting the concentrated diluent A stored in the first container 700 to a predetermined concentration in the dilution tank DT and supplying it to the measurement unit 10, and continuing the supply of the diluent from the dilution tank DT even while the first container 700 is being replaced.

FIG. 29 is a schematic diagram showing an example configuration of the supply unit 40 in Embodiment 4. For simplification of description, FIG. 29 only shows the fluid circuit for supplying the concentrated diluent A to the measurement unit 10 within the supply unit 40. The reagent storage unit 50 includes a first container 700 as a container for storing the concentrated diluent A. The supply unit 40 includes a dilution tank DT. The first container 700 is fluidically connected to the dilution tank DT via an air bubble sensor BS. As described with reference to FIG. 5, the first tube 51a extending from the dilution tank DT is inserted into the opening of the container, fluidically connecting the first container 700 and the dilution tank DT. Although not shown in the figure, a reserve tank may be provided between the first container 700 and the dilution tank DT.

The tank T stores water. The tank T is fluidically connected to the dilution tank DT via a tube. Water is drawn from the bottom of the tank T to supply diluent to the dilution tank DT. The water stored in the tank T may be, for example, water supplied from a water supply outside the measurement apparatus or water supplied via the water purification apparatus 550 within the measurement apparatus.

The dilution tank DT includes, for example, a float sensor FS. As mentioned above, the float sensor FS detects the remaining volume of reagent in the reserve tank. For example, when the float sensor FS of the dilution tank DT detects a decrease in the remaining reagent volume, the supply unit 40 controls the measurement control unit 852 (see FIG. 18) to drive the pump 57 and supply a predetermined amount of concentrated diluent A stored in the first container 700 and a predetermined amount of water to the dilution tank DT. In the dilution tank DT, the concentrated diluent A and water are mixed to prepare Diluent A of an appropriate concentration. The dilution tank DT and the measurement unit 10 are connected via a tube. Diluent A is supplied to the measurement unit 10 by drawing Diluent A from the bottom of the dilution tank DT in response to the measurement operation by the measurement unit 10.

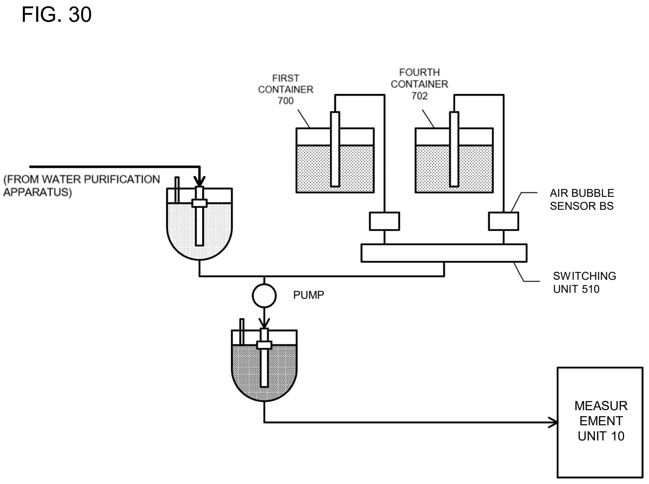

<Embodiment 5> The measurement apparatus of Embodiment 5 is a measurement apparatus that includes the switching function for concentrated diluent reagent in addition to the dilution tank DT provided in the measurement apparatus of Embodiment 4. FIG. 30 is a schematic diagram showing an example configuration of the supply unit 40 in Embodiment 5. The supply unit 40 of Embodiment 5 additionally includes a switching unit 510 and a fourth container 702 switchable with the first container 700, compared to the supply unit 40 of Embodiment 4.

The configuration of the dilution tank DT is the same as described in Embodiment 4. As described in Embodiment 2, the switching unit 510 includes a valve for switching the supply source of the concentrated diluent A between the first container 700 and the fourth container 702. The switching unit 510 can switch between a first flow path that fluidically connects the first container 700 and the dilution tank DT and a second flow path that fluidically connects the fourth container 702 and the dilution tank DT. Other configurations are the same as described in Embodiments 2 and 4.

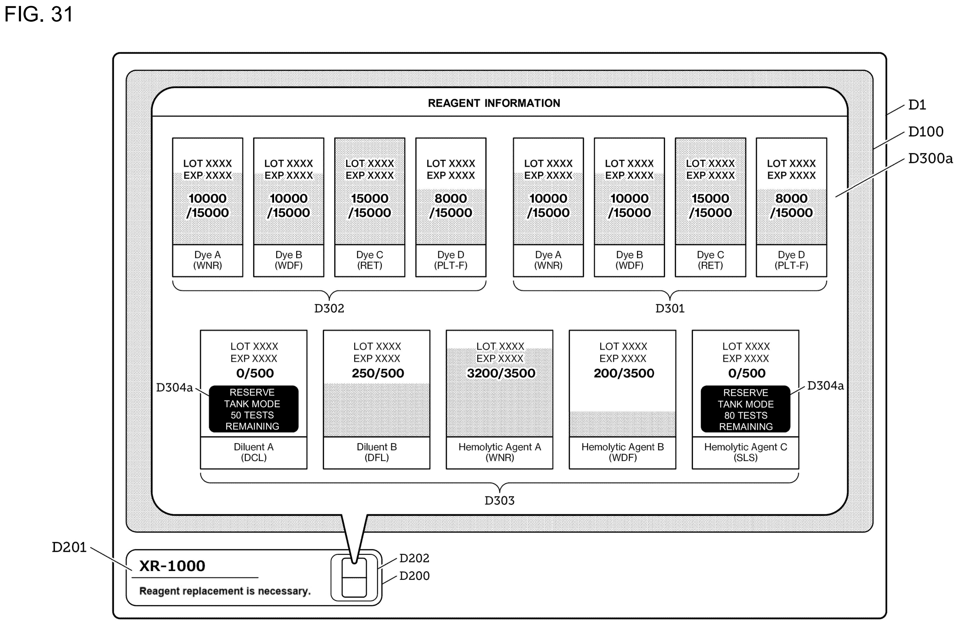

<Other Configuration Examples> FIG. 31 is a modified example of the reagent information screen in Embodiment 1. In Embodiment 1 described above, when the first container 700 and the second container 800 run out of reagent and the supply source is switched to the reserve tank, a replacement alarm D304 is displayed. In the modified example, in addition to or instead of the display prompting replacement, the remaining volume in the reserve tank may be displayed. For example, in FIG. 31, a graphic D304a showing the remaining volume in the reserve tank is displayed instead of the replacement alarm D304. The graphic D304a displays, for example, an indication that the reagent supply mode is the reserve tank mode (referring to the state where the supply source has switched to the reserve tank), and the remaining volume in the reserve tank is displayed in real-time in the format of the number of measurable tests. Such a display allows the user to understand that reagent supply is continuing from the reserve tank. Furthermore, by understanding the remaining volume in the reserve tank, the user can know by when the reagent must be replaced to avoid stopping the measurement, thereby supporting the laboratory operation for realizing continuous loading. Although FIG. 31 shows an example of the screen displayed on the display 70, the indication of the reserve tank mode and the remaining volume in the reserve tank may also be displayed on the display screen of the mobile terminal illustrated in FIG. 18.

The present disclosure includes following items:

Item 1: A measurement apparatus operable to measure a blood sample, comprising: a reagent storage unit configured to accommodate a first container storing a diluent to dilute a blood sample and a second container storing a hemolytic agent to lyse red blood cells contained in the blood sample; a transport unit arranged above the reagent storage unit, wherein the transport unit includes a first region and a second region on which the blood sample is placed and the transport unit is configured to transport the placed blood sample; a measurement unit configured to measure the blood sample transported from the first region and to discharge the measured blood sample to the second region; a supply unit configured to supply the diluent and the hemolytic agent to the measurement unit; and a control unit configured to control the transport unit, the measurement unit, and the supply unit, wherein the measurement unit includes: a third container storing a staining solution to stain the blood sample; a first chamber configured for mixing the diluent supplied from the first container and the blood sample to prepare a first measurement sample; a second chamber configured for mixing the hemolytic agent supplied from the second container, the blood sample, and the staining solution to prepare a second measurement sample; and a measuring section including an electrical signal detector to interrogate the first measurement sample to obtain an electrical signal and an optical signal detector to interrogate the second measurement sample to obtain an optical signal, wherein the measurement unit is configured to obtain the electrical signal and the optical signal for 300 to 500 blood samples per hour according to a measurement order for each of the blood samples, the measurement order including: (1) a first measurement item including red blood cell count, white blood cell count, hemoglobin concentration, hematocrit value, mean corpuscular volume, mean corpuscular hemoglobin, mean corpuscular hemoglobin concentration, and platelet count; and (2) a second measurement item of five-part white blood cell differential counting, and to provide a measurement result based on the obtained electrical signal and the optical signal, and wherein the supply unit is configured to continue the supply of the diluent while the first container is being replaced and to continue the supply of the hemolytic agent while the second container is being replaced.

Item 2: The measurement apparatus according to item 1, wherein the supply unit is configured to: continue the supply of the diluent in response to the diluent stored in the first container being at or below a predetermined first remaining amount, and continue the supply of the hemolytic agent in response to the hemolytic agent stored in the second container being at or below a predetermined second remaining amount.

Item 3: The measurement apparatus according to item 1, wherein the supply unit is configured to: continue the supply of the diluent during a disconnection between the measurement unit and the first container having the remaining amount equal to or below the predetermined first remaining amount, and continue the supply of the hemolytic agent during a disconnection between the measurement unit and the second container having the remaining amount equal to or below the predetermined second remaining amount.

Item 4: The measurement apparatus according to item 1, wherein the reagent storage unit includes an openable and closable drawer or door, and the supply unit is configured to: continue the supply of the diluent while the drawer or the door is open to replace the first container, and continue the supply of the hemolytic agent while the drawer or the door is open to replace the second container.

Item 5: The measurement apparatus according to item 1, wherein the control unit is configured to control the measurement unit to stop preparation of the first measurement sample and the second measurement sample and measurement by the measuring section in response to the remaining amount of the staining solution in the third container being at or below a predetermined amount.

Item 6: The measurement apparatus according to item 1, wherein the first container is configured to accommodate a first amount of the diluent, the third container is configured to accommodate a second amount of the staining solution, and the number of measurement samples measurable by the measurement unit using the second amount of the staining solution is greater than the number of measurement samples measurable using the first amount of the diluent accommodated in the first container.

Item 7: The measurement apparatus according to item 6, wherein the second container is configured to accommodate a third amount of the hemolytic agent, and the number of measurement samples measurable by the measurement unit using the second amount of the staining solution is greater than the number of measurement samples measurable using the third amount of the hemolytic agent accommodated in the second container.

Item 8: The measurement apparatus according to item 6, wherein the third container accommodates an amount of the staining solution capable of preparing the second measurement sample for 5,000 to 30,000 test samples.

Item 9: The measurement apparatus according to item 8, wherein the first container accommodates an amount of the diluent capable of preparing the first measurement sample and the second measurement sample for 300 to 1,500 test samples.

Item 10: The measurement apparatus according to item 1, wherein the measurement unit includes a plurality of third containers, and the plurality of third containers respectively store the staining solution used for the first measurement item and the staining solution used for the second measurement item.

Item 11: The measurement apparatus according to item 1, wherein the supply unit supplies both the diluent stored in the first container and the hemolytic agent stored in the second container to the measurement unit upon receiving a measurement order including the first measurement item, and supplies both the diluent stored in the first container and the hemolytic agent stored in the second container to the measurement unit even upon receiving a measurement order including the first measurement item and the second measurement item.

Item 12: The measurement apparatus according to item 1, wherein the measurement unit includes an aspiration tube to aspirate the staining solution, and the control unit controls the measurement unit to stop the measurement in response to the aspiration tube being pulled out from the third container.

Item 13: The measurement apparatus according to item 1, further comprising a first reserve tank storing the diluent accommodated in the first container, wherein the supply unit supplies the diluent stored in the first reserve tank to the measurement unit in a state where the supply of the diluent from the first container is stopped.

Item 14: The measurement apparatus according to item 13, wherein the first reserve tank is configured to store an amount of the diluent substantially corresponding to measurements of 200 blood samples based on a measurement order for a complete blood count and five-part white blood cell differential counting.

Item 15: The measurement apparatus according to item 13, further comprising a second reserve tank storing the hemolytic agent accommodated in the second container, wherein the supply unit supplies the hemolytic agent stored in the second reserve tank to the measurement unit in a state where the supply of the hemolytic agent from the second container is stopped.

Item 16: The measurement apparatus according to item 13, wherein the amount of the diluent storable in the first reserve tank is less than the amount of the diluent accommodatable in the first container.

Item 17: The measurement apparatus according to item 13, wherein the amount of the hemolytic agent storable in the second reserve tank is less than the amount of the hemolytic agent accommodatable in the second container.

Item 18: The measurement apparatus according to item 13, wherein the measurement unit is capable of measuring a test sample using the diluent supplied from the first reserve tank, and the supply unit supplies the diluent from the first container to the first reserve tank while supplying the diluent to the measurement unit from the first reserve tank.

Item 19: The measurement apparatus according to item 13, wherein the supply unit supplies the diluent from the first container to the first reserve tank according to the consumption amount of the diluent in the first reserve tank.

Item 20: The measurement apparatus according to item 13, wherein the control unit stops measurement of a new blood sample in a state where the amount of the diluent accommodated in the first container is at or below a predetermined amount and the amount of the diluent stored in the first reserve tank is at or below a predetermined amount.

Item 21: The measurement apparatus according to item 20, wherein the control unit detects the remaining amount in the first container based on either (1) the number of measurements using the diluent in the first container or (2) a signal from a sensor that detects the remaining amount in the first container.

Item 22: The measurement apparatus according to item 1, wherein the reagent storage unit is further configured to accommodate a fourth container storing the diluent and a fifth container storing the hemolytic agent, and the supply unit is configured to:

continue the supply of the diluent from the fourth container upon failure of supply from the first container, and continue the supply of the hemolytic agent from the fifth container upon failure of supply from the second container.

Item 23: The measurement apparatus according to item 1, wherein the reagent storage unit is further configured to accommodate a fourth container storing the diluent, and the supply unit is configured to automatically switch the supply source of the diluent from the first container to the fourth container in response to the remaining amount in the first container becoming at or below a predetermined amount.

Item 24: The measurement apparatus according to item 1, wherein the reagent storage unit is further configured to accommodate a fourth container storing the diluent, and the supply unit is configured to: automatically switch the supply source of the diluent from the first container to the fourth container in response to the remaining amount in the first container becoming at or below a predetermined amount while the supply source of the diluent is the first container, and automatically switch the supply source of the diluent from the fourth container to the first container in response to the remaining amount in the fourth container becoming at or below a predetermined amount while the supply source of the diluent is the fourth container.

Item 25: The measurement apparatus according to item 1, further comprising a first tube inserted into the first container and a second tube inserted into the second container, wherein the first tube and the second tube are each identifiable for the container into which they should be inserted.

Item 26: The measurement apparatus according to item 22, wherein the reagent storage unit is further configured to accommodate a fifth container storing the hemolytic agent, and the supply unit is configured to automatically switch the supply source of the hemolytic agent from the second container to the fifth container in response to the remaining amount in the second container becoming at or below a predetermined amount.

Item 27: The measurement apparatus according to item 1, further comprising a display unit, wherein the control unit causes the display unit to display the remaining amount of the diluent in the first container and the remaining amount of the hemolytic agent in the second container.

Item 28: The measurement apparatus according to item 26, wherein the control unit causes the display unit to output a first alarm to prompt replacement of the first container in a state where the remaining amount of the diluent accommodated in the first container is at or below a predetermined amount.

Item 29: The measurement apparatus according to item 26, wherein the control unit causes the display unit to output a second alarm to prompt replacement of the second container in a state where the remaining amount of the hemolytic agent accommodated in the second container is at or below a predetermined amount.

Item 30: The measurement apparatus according to item 1, wherein the control unit causes a terminal capable of communicating with the control unit to output a first alarm to prompt replacement of the first container.

Item 31: The measurement apparatus according to item 1, wherein the control unit causes a terminal capable of communicating with the control unit to output a second alarm to prompt replacement of the second container.

Item 32: The measurement apparatus according to item 1, wherein the reagent storage unit is further configured to accommodate a fourth container storing the diluent, the supply unit is configured to continue the supply of the diluent from the fourth container upon failure of supply from the first container, and the apparatus further includes a reading unit to read reagent information from a reagent container, wherein the control unit overwrites the reagent information of the first container with the reagent information of a new container storing the diluent in response to the reagent information of the new container being read by the reading unit.

Item 33: The measurement apparatus according to item 1, wherein the reagent storage unit is further configured to accommodate a fifth container storing the diluent, the supply unit is configured to continue the supply of the diluent from the fifth container upon failure of supply from the second container, and the apparatus further includes a reading unit to read reagent information from a reagent container, wherein the control unit overwrites the reagent information of the second container with the reagent information of a new container storing the diluent in response to the reagent information of the new container being read by the reading unit.

Item 34: The measurement apparatus according to item 1, wherein the supply unit includes a dilution device to dilute the diluent accommodated in the first container at a predetermined dilution ratio, and supplies the diluent diluted at the predetermined dilution ratio by the dilution device to the measurement unit.

Item 35: The measurement apparatus according to item 34, wherein the dilution device dilutes the diluent by mixing water supplied from outside the measurement apparatus and the diluent supplied from the first container, and the supply unit supplies the diluted diluent to the measurement unit.

Item 36: The measurement apparatus according to item 34, wherein the reagent storage unit is further configured to accommodate a fourth container storing the diluent, and the supply unit is configured to: dilute the diluent accommodated in the fourth container by the dilution device upon failure of supply from the first container, and supply the diluent diluted at a predetermined dilution ratio by the dilution device to the measurement unit.

Item 37: The measurement apparatus according to item 1, wherein the reagent storage unit is configured to accommodate 20 L of the diluent and 10 L of the hemolytic agent, the measurement unit includes a staining solution storage unit, and the staining solution storage unit is configured to accommodate 10 mL of the staining solution.

Item 38: The measurement apparatus according to item 1, wherein the supply unit supplies the diluent to the measurement unit such that the measurement unit arranged above the reagent storage unit uses the diluent accommodated in the first container exclusively.

Item 39: The measurement apparatus according to item 1, wherein the supply unit supplies the hemolytic agent to the measurement unit such that the measurement unit arranged above the reagent storage unit uses the hemolytic agent accommodated in the second container exclusively.

Item 40: The measurement apparatus according to item 1, wherein the supply unit includes a flow path to exclusively supply the diluent accommodated in the first container to the measurement unit arranged above the reagent storage unit.

Item 41: The measurement apparatus according to item 1, wherein the supply unit includes a flow path to exclusively supply the hemolytic agent accommodated in the second container to the measurement unit arranged above the reagent storage unit.

Item 42: The measurement apparatus according to item 1, wherein the control unit manages the remaining amount of the diluent used exclusively by the measurement unit arranged above the reagent storage unit.

Item 43: The measurement apparatus according to item 1, wherein the control unit manages the remaining amount of the hemolytic agent used exclusively by the measurement unit arranged above the reagent storage unit.

Item 44: A measurement apparatus, comprising: a reagent storage unit configured to accommodate a first container storing a diluent to dilute a blood sample and a second container storing a hemolytic agent to lyse red blood cells contained in the blood sample; a transport unit arranged above the reagent storage unit, wherein the transport unit includes a first region and a second region on which the blood sample is placed and the transport unit is configured to transport the placed blood sample; a measurement unit configured to measure the blood sample transported from the first region and to discharge the measured blood sample to the second region, wherein the measurement unit is arranged above the reagent storage unit; a supply unit configured to supply the diluent and the hemolytic agent to the measurement unit; and a control unit configured to control the transport unit, the measurement unit, and the supply unit, wherein the measurement unit includes: a third container configured to accommodate a staining solution to stain the blood sample; a first chamber configured for mixing the diluent supplied from the first container and the blood sample to prepare a first measurement sample; a second chamber configured for mixing the hemolytic agent supplied from the second container, the blood sample, and the staining solution to prepare a second measurement sample; and a measuring section including an electrical signal detector to measure an electrical signal regarding the first measurement sample and an optical signal detector to measure an optical signal regarding the second measurement sample, wherein the measurement unit is configured to prepare the first measurement sample and the second measurement sample to obtain the electrical signal and the optical signal in response to a measurement order including: (1) a first measurement item including red blood cell count, white blood cell count, hemoglobin concentration, hematocrit value, mean corpuscular volume, mean corpuscular hemoglobin, mean corpuscular hemoglobin concentration, and platelet count; and (2) a second measurement item of five-part white blood cell differential counting, and to provide a measurement result based on the obtained electrical signal and the optical signal, and wherein the supply unit is configured to supply the diluent to the measurement unit such that the measurement unit arranged above the reagent storage unit uses the diluent accommodated in the first container exclusively, to continue the supply of the diluent while the first container is being replaced, to supply the hemolytic agent to the measurement unit such that the measurement unit arranged above the reagent storage unit uses the hemolytic agent accommodated in the second container exclusively, and to continue the supply of the hemolytic agent while the second container is being replaced.

Item 45: The measurement apparatus according to item 44, wherein the supply unit includes a flow path to exclusively supply the diluent accommodated in the first container to the measurement unit arranged above the reagent storage unit.

Item 46: The measurement apparatus according to item 44, wherein the supply unit includes a flow path to exclusively supply the hemolytic agent accommodated in the second container to the measurement unit arranged above the reagent storage unit.

Item 47: The measurement apparatus according to item 44, wherein the control unit manages the remaining amount of the diluent used exclusively by the measurement unit arranged above the reagent storage unit.

Item 48: The measurement apparatus according to item 44, wherein the control unit manages the remaining amount of the hemolytic agent used exclusively by the measurement unit arranged above the reagent storage unit.

**Claims**

1. A measurement apparatus operable to measure a blood sample, comprising:

    a reagent storage unit configured to accommodate a first container storing a diluent to dilute a blood sample and a second container storing a hemolytic agent to lyse red blood cells contained in the blood sample;
    a transport unit arranged above the reagent storage unit, wherein the transport unit includes a first region and a second region on which the blood sample is placed and the transport unit is configured to transport the placed blood sample;
    a measurement unit configured to measure the blood sample transported from the first region and to discharge the measured blood sample to the second region;
    a supply unit configured to supply the diluent and the hemolytic agent to the measurement unit; and
    a control unit configured to control the transport unit, the measurement unit, and the supply unit, wherein the measurement unit includes:

a third container storing a staining solution to stain the blood sample;

a first chamber configured for mixing the diluent supplied from the first container and the blood sample to prepare a first measurement sample;

a second chamber configured for mixing the hemolytic agent supplied from the second container, the blood sample, and the staining solution to prepare a second measurement sample; and

a measuring section including an electrical signal detector to interrogate the first measurement sample to obtain an electrical signal and an optical signal detector to interrogate the second measurement sample to obtain an optical signal,

wherein the measurement unit is configured to obtain the electrical signal and the optical signal for 300 to 500 blood samples per hour according to a measurement order for each of the blood samples, the measurement order including:

> (1) a first measurement item including red blood cell count, white blood cell count, hemoglobin concentration, hematocrit value, mean corpuscular volume, mean corpuscular hemoglobin, mean corpuscular hemoglobin concentration, and platelet count; and
> (2) a second measurement item of five-part white blood cell differential counting, and to provide a measurement result based on the obtained electrical signal and the optical signal, and

wherein the supply unit is configured to continue the supply of the diluent while the first container is being replaced and to continue the supply of the hemolytic agent while the second container is being replaced.

2. The measurement apparatus according to claim 1, wherein the supply unit is configured to:

continue the supply of the diluent during a disconnection between the measurement unit and the first container having the remaining amount equal to or below the predetermined first remaining amount, and

continue the supply of the hemolytic agent during a disconnection between the measurement unit and the second container having the remaining amount equal to or below the predetermined second remaining amount.

3. The measurement apparatus according to claim 1 or 2, wherein the reagent storage unit includes an openable and closable drawer or door, and the supply unit is configured to:

continue the supply of the diluent while the drawer or the door is open to replace the first container, and

continue the supply of the hemolytic agent while the drawer or the door is open to replace the second container.

4. The measurement apparatus according to any one of claims 1 to 3, wherein the control unit is configured to control the measurement unit to stop preparation of the first measurement sample and the second measurement sample and measurement by the measuring section in response to the remaining amount of the staining solution in the third container being at or below a predetermined amount.

5. The measurement apparatus according to any one of claims 1 to 4, wherein the first container is configured to accommodate a first amount of the diluent, the third container is configured to accommodate a second amount of the staining solution, and the number of blood samples measurable by the measurement unit using the second amount of the staining solution is greater than the number of blood samples measurable using the first amount of the diluent accommodated in the first container.

6. The measurement apparatus according to any one of claims 1 to 5, wherein the measurement unit includes an aspiration tube to aspirate the staining solution, and the control unit controls the measurement unit to stop the measurement in response to the aspiration tube being pulled out from the third container.

7. The measurement apparatus according to any one of claims 1 to 6, further comprising a first reserve tank storing the diluent accommodated in the first container, wherein the supply unit supplies the diluent stored in the first reserve tank to the measurement unit in a state where the supply of the diluent from the first container is stopped.

8. The measurement apparatus according to claim 7, wherein the first reserve tank is configured to store an amount of the diluent substantially corresponding to measurements of 200 blood samples based on a measurement order for a complete blood count and five-part white blood cell differential counting.

9. The measurement apparatus according to claim 7 or 8, wherein the measurement unit is capable of measuring a blood sample using the diluent supplied from the first reserve tank, and the supply unit supplies the diluent from the first container to the first reserve tank while supplying the diluent to the measurement unit from the first reserve tank.

10. The measurement apparatus according to any one of claims 7 to 9, wherein the control unit stops measurement of a new blood sample in a state where the amount of the diluent accommodated in the first container is at or below a predetermined amount and the amount of the diluent stored in the first reserve tank is at or below a predetermined amount.

11. The measurement apparatus according to any one of claims 1 to 10, wherein the reagent storage unit is further configured to accommodate a fourth container storing the diluent and a fifth container storing the hemolytic agent, and the supply unit is configured to:

continue the supply of the diluent from the fourth container upon failure of supply from the first container, and
continue the supply of the hemolytic agent from the fifth container upon failure of supply from the second container.

12. The measurement apparatus according to any one of claims 1 to 11, wherein the reagent storage unit is further configured to accommodate a fourth container storing the diluent, and the supply unit is configured to automatically switch the supply source of the diluent from the first container to the fourth container in response to the remaining amount in the first container becoming at or below a predetermined amount.

13. The measurement apparatus according to any one of claims 1 to 12, wherein the reagent storage unit is further configured to accommodate a fourth container storing the diluent, and the supply unit is configured to:
automatically switch the supply source of the diluent from the first container to the fourth container in response to the remaining amount in the first container becoming at or below a predetermined amount while the supply source of the diluent is the first container, and automatically switch the supply source of the diluent from the fourth container to the first container in response to the remaining amount in the fourth container becoming at or below a predetermined amount while the supply source of the diluent is the fourth container.

14. The measurement apparatus according to any one of claims 1 to 13, further comprising a first tube inserted into the first container and a second tube inserted into the second container, wherein the first tube and the second tube are each identifiable for the container into which they should be inserted.

15. The measurement apparatus according to any one of claims 1 to 14, wherein the reagent storage unit is further configured to accommodate a fifth container storing the diluent, the supply unit is configured to continue the supply of the diluent from the fifth container upon failure of supply from the second container, and the apparatus further includes a reading unit to read reagent information from a reagent container, wherein the control unit overwrites the reagent information of the second container with the reagent information of a new container storing the diluent in response to the reagent information of the new container being read by the reading unit.

16. The measurement apparatus according to any one of claims 1 to 15, wherein the supply unit includes a dilution device to dilute the diluent accommodated in the first container at a predetermined dilution ratio, and supplies the diluent diluted at the predetermined dilution ratio by the dilution device to the measurement unit.

17. The measurement apparatus according to any one of claims 1 to 16, wherein the supply unit supplies the diluent to the measurement unit such that the measurement unit arranged above the reagent storage unit uses the diluent accommodated in the first container exclusively.

FIG. 1

MEASUREMENT APPARATUS 1

MEASUREMENT UNIT 10

THIRD CONTAINER 900

FIRST MEASUREMENT UNIT 11

SECOND MEASUREMENT UNIT 12

DISPLAY 70

SAMPLE CONTAINER 110

RACK R

TRANSPORT UNIT 30

CONTROL UNIT 200

WAGON 60

SECOND CONTAINER 800

FIRST CONTAINER 700

SUPPLY UNIT 40

REAGENT STORAGE UNIT 50

WATER PURIFICATION APPARATUS 550

EP 4 782 842 A1

FIG. 2

FIG. 3

FIG. 4

MEASUREMENT UNIT 10

OPENING/CLOSING UNIT 61

FIRST CONTAINER 700

WAGON 60

SECOND CONTAINER 80

SECOND CONTAINER 80

DRAWER 52

DRAWER 52

REAGENT STORAGE UNIT 50

SECOND CONTAINER 800

DRAWER 52

## FIG. 5

TUBE 51

SUPPLY UNIT 40

SECOND TUBE 51b

FIRST TUBE 51a

CONNECTOR 53

FIRST CONTAINER 700

OUTER BOX 58

OPENING 54

FIG. 6

OPENING/
CLOSING
UNIT 111

STAINING SOLUTION
STORAGE UNIT 112

FIG. 7

SUPPLY UNIT 40

FS

RT1

RT

RT2

RT3

RT4

RT5

BS

MEASURE
MENT
UNIT 10

PUMP 57

700
(DILUENT A)

701
(DILUENT B)

800
(HEMOLYTIC
AGENT A)

801
(HEMOLYTIC
AGENT B)

802
(HEMOLYTIC
AGENT C)

EP 4 782 842 A1

39

FIG. 8

STAINING SOLUTION
STORAGE UNIT 112

CHAMBER C21, C22

56

CHAMBER C23

CHAMBER C24

55

900
(STAINING SOLUTION A)

901
(STAINING SOLUTION B)

902
(STAINING SOLUTION C)

903
(STAINING SOLUTION D)

EP 4 782 842 A1

FIG. 9

| REAGENT TYPE | VOLUME | NUMBER OF MEASURABLE TESTS (COUNT) |
|---|---|---|
| DILUENT A | 10L | 500 |
| DILUENT B | 10L | 500 |
| HEMOLYTIC AGENT A | 5L | 3500 |
| HEMOLYTIC AGENT B | 5L | 3500 |
| HEMOLYTIC AGENT C | 5L | 3500 |
| STAINING SOLUTION A | 300mL | 15000 |
| STAINING SOLUTION B | 300mL | 15000 |
| STAINING SOLUTION C | 300mL | 15000 |
| STAINING SOLUTION D | 300mL | 15000 |

FIG. 10

FIG. 11

EP 4 782 842 A1

FIG. 12

EP 4 782 842 A1

# FIG. 13

**FLOW CELL OF OPTICAL
MEASUREMENT UNIT**

LASER LIGHT

44

381

65

43

43a

41

42

## FIG. 14

**FLOW CELL OF ELECTRICAL
MEASUREMENT UNIT**

66
154
155
153
152
151
382

**HGB MEASUREMENT UNIT**

383
383a
383c
383b

FIG. 15

FIG. 16

TO HOST COMPUTER

200

**CONTROL UNIT**

850

CONTROLLER

85

ANALYSIS UNIT

852

MEASUREMENT CONTROL UNIT

861

STORAGE

853

DISPLAY

863

COMMUNICATION UNIT

854

INPUT PART

862

COMMUNICATION UNIT

TO MEASUREMENT UNIT

30

**TRANSPORT UNIT**

34

READING UNIT

251

MECHANISM UNIT

252

COMMUNICATION UNIT

FIG. 17

2 SECONDS
APPROX. 24 SECOND CYCLE
OVERLAP PERIOD OF WDF MEASUREMENT

HGB MEASUREMENT
HGB PREPARATION
RBC PREPARATION
RBC MEASUREMENT
WDF PREPARATION
WDF MEASUREMENT
WNR PREPARATION
WNR MEASUREMENT

HGB PREPARATION
HGB MEASUREMENT
RBC PREPARATION
RBC MEASUREMENT
WDF PREPARATION
WDF MEASUREMENT
WNR PREPARATION
WNR MEASUREMENT

EP 4 782 842 A1

## FIG. 18

**EXAMPLE OF DILUENT SUPPLY**

```
                                              S1
        ┌──────────────────────────────┐
        │   SUPPLY DILUENT FROM FIRST   │
   ┌───▶│  CONTAINER UNTIL REMAINING    │
   │    │     VOLUME FALLS BELOW        │
   │    │    PREDETERMINED AMOUNT       │
   │    └──────────────────────────────┘
   │                   │              S2
   │    ┌──────────────────────────────┐
   │    │      REPLACEMENT ALARM        │
   │    └──────────────────────────────┘
   │                   │              S3
   │    ┌──────────────────────────────┐
   │    │       SUPPLY DILUENT          │
   │    │     FROM RESERVE TANK         │
   │    └──────────────────────────────┘
   │                   │
   │                   │
   │              S4   ▼
   │    ┌──────────────────────┐
   │    │      REAGENT          │  NO
   │    │   REPLACEMENT ?       │──────────────┐
   │    └──────────────────────┘               │
   │            │ YES                           ▼
   │            │                         ┌─────────────────────────┐  S5
   │            │                         │  REMAINING VOLUME IN     │
   │            │                         │    RESERVE TANK?         │── YES
   │            │                         └─────────────────────────┘
   │            │                               │ NO
   │     S7     ▼                               ▼               S6
   │    ┌──────────────────────┐         ┌─────────────────────────┐
   │    │   REGISTRATION OF     │         │         STOP            │
   │    │  REAGENT INFORMATION/ │         └─────────────────────────┘
   │    │    ALARM RELEASE      │
   │    └──────────────────────┘
   │            │
   └────────────┘
```

# FIG. 19

REAGENT INFORMATION

| 2023/6/1 | 2023/6/1 | 2023/6/1 | 2023/6/1 | 2023/6/1 | 2023/6/1 | 2023/6/1 | 2023/6/1 |
|---|---|---|---|---|---|---|---|
| LOT REMAINING | LOT REMAINING | LOT REMAINING | LOT REMAINING | LOT REMAINING | LOT REMAINING | LOT REMAINING | LOT REMAINING |
| Dye A (WNR) | Dye B (WDF) | Dye C (RET) | Dye D (PLT-F) | Dye A (WNR) | Dye B (WDF) | Dye C (RET) | Dye D (PLT-F) |

D302    D301

| LOT XXXX 0 TESTS REMAINING ! REPLACE | LOT XXXX 150 TESTS REMAINING | LOT XXXX 200 TESTS REMAINING | LOT XXXX 30 TESTS REMAINING | LOT XXXX 0 TESTS REMAINING ! REPLACE |
|---|---|---|---|---|
| Diluent A (DCL) | Diluent B (DFL) | Hemolytic Agent A (WNR) | Hemolytic Agent B (WDF) | Hemolytic Agent C (SLS) |

D304    D304

D303

D201

**XR-1000**

**Reagent replacement is necessary.**

D202
D200

D1
D100
D300

EP 4 782 842 A1

FIG. 20

1000

DILUENT A HAS RUN
OUT IN XR-1000

D100

XR-1000
ID:XXXXXX

D101

Diluent A
0/500

D120

Diluent B
150/500

Hemolytic Agent A
150/500

D110

Hemolytic Agent B
150/500

Hemolytic Agent C
0/500

EP 4 782 842 A1

FIG. 21

(a)

D330

REPLACE DILUENT A AND
PUSH COMPLETION BUTTON

COMPLETE

(b)

D331

REPLACE DILUENT A AND INPUT
REAGENT INFORMATION OF NEW
DILUENT A

LOT
NUMBER        XXXXX

EXPIRATION
DATE          XXXXX

REGISTER

EP 4 782 842 A1

FIG. 22

(a)

**EXAMPLE OF
STAINING SOLUTION 1**

S1
REMAINING VOLUME IS
BELOW PREDETERMINED
AMOUNT ?
NO

YES

S2
REPLACEMENT
ALARM

S3
STOP MEASUREMENT UNIT

S4
REAGENT REPLACEMENT ?
NO

YES

S5
REGISTRATION OF REAGENT
INFORMATION / ALARM
RELEASE

(b)

**EXAMPLE OF
STAINING SOLUTION 2**

S1
REMAINING VOLUME IS
AT FIRST LEVEL ?
NO

YES

S2
REPLACEMENT
ALARM

S3
REMAINING VOLUME IS
AT SECOND LEVEL?
NO

YES

S4
STOP MEASUREMENT UNIT

S5
REAGENT REPLACEMENT ?
NO

YES

S6
ALARM RELEASE

54

FIG. 23

FIG. 24

REAGENT INFORMATION

| 2023/6/1 | 2023/6/1 | 2023/6/1 | 2023/6/1 |
|---|---|---|---|
| **LOT XXX** **EXP XXX** 12000/ 15000 | **LOT XXX** **EXP XXX** 8000/ 15000 | **LOT XXX** **EXP XXX** 15000/ 15000 | **LOT XXX** **EXP XXX** 15000/ 15000 |
| Dye A (WNR) | Dye B (WDF) | Dye C (RET) | Dye D (PLT-F) |

| **DA1** | **DA2** | **DB1** | **DB2** | **HA1** | **HA2** | **HB1** | **HB2** | **HC1** | **HC2** |
|---|---|---|---|---|---|---|---|---|---|
| LOT XXX EXP XXX 400/ 500 IN USE | LOT XXX EXP XXX 0/ 500 ❗ REPLACE | LOT XXX EXP XXX 500/ 500 IN USE | LOT XXX EXP XXX 0/ 500 ❗ REPLACE | LOT XXX EXP XXX 3500/ 3500 | LOT XXX EXP XXX 1100/ 3500 IN USE | LOT XXX EXP XXX 1100/ 3500 IN USE | LOT XXX EXP XXX 3500/ 3500 | LOT XXX EXP XXX 3500/ 3500 IN USE | LOT XXX EXP XXX 0/ 3500 ❗ REPLACE |
| Diluent A (DCL) | | Diluent B (DFL) | | Hemolytic Agent A (WNR) | | Hemolytic Agent B (WDF) | | Hemolytic Agent C (SLS) | |

D1
D100
D300

D201

**XR-1000**

Reagent replacement is necessary.

D202
D200

EP 4 782 842 A1

56

FIG. 25

EP 4 782 842 A1

FIG. 26

**EXAMPLE OF DILUENT**

```
                                              S1
┌─────────────────────────────┐
│ SUPPLY DILUENT FROM CONTAINER │
│ IN USE UNTIL REMAINING VOLUME │
│ FALLS BELOW PREDETERMINED     │
│ AMOUNT                        │
└─────────────────────────────┘
                                              S2
┌─────────────────────────────┐
│ REPLACEMENT ALARM             │
└─────────────────────────────┘
                                              S3
       BACKUP CONTAINER              NO                          S5
       PRESENT ?                                    ┌──────────────────┐
                                                    │ STOP             │
           YES                                      └──────────────────┘
                                              S4
┌─────────────────────────────┐
│ SWITCH TO BACKUP              │
│ CONTAINER                     │
└─────────────────────────────┘
                                              S6
       REAGENT                       NO
       REPLACEMENT ?

           YES
                                              S7
┌─────────────────────────────┐
│ REGISTRATION OF               │
│ REAGENT INFORMATION /          │
│ ALARM RELEASE                 │
└─────────────────────────────┘
```

58

# FIG. 27

FIG. 28

**IN THE CASE OF DILUENT**

S1 — SUPPLY DILUENT FROM CONTAINER IN USE TO MEASUREMENT UNIT VIA RESERVE TANK UNTIL REMAINING VOLUME IS BELOW PREDETERMINED AMOUNT

S2 — CONTAINER REPLACEMENT ALARM

S3 — BACKUP PRESENT ?
- YES
- NO

S7 — CONTINUE DILUENT SUPPLY FROM RESERVE TANK

S4 — SWITCH TO BACKUP CONTAINER

S5 — REAGENT REPLACEMENT ?
- YES
- NO

S8 — REAGENT REPLACEMENT ?
- YES
- NO

S9 — REMAINING VOLUME IN RESERVE TANK PRESENT?
- YES
- NO

S6 — REGISTRATIO OF REAGENT INFORMATION / ALARM RELEASE

STOP

FIG. 29

RO WATER
(FROM RO DEVICE)

TANK T

AIR BUBBLE
SENSOR BS

700

CONCENTRAT
ED DILUENT

PUMP 57

FS

DT

MEASUR
EMENT
UNIT 10

FIG. 30

## FIG. 31

REAGENT INFORMATION

| LOT XXXX EXP XXXX 10000/15000 Dye A (WNR) | LOT XXXX EXP XXXX 10000/15000 Dye B (WDF) | LOT XXXX EXP XXXX 15000/15000 Dye C (RET) | LOT XXXX EXP XXXX 8000/15000 Dye D (PLT-F) | LOT XXXX EXP XXXX 10000/15000 Dye A (WNR) | LOT XXXX EXP XXXX 10000/15000 Dye B (WDF) | LOT XXXX EXP XXXX 15000/15000 Dye C (RET) | LOT XXXX EXP XXXX 8000/15000 Dye D (PLT-F) |

D302    D301

| LOT XXXX EXP XXXX 0/500 RESERVE TANK MODE 50 TESTS REMAINING Diluent A (DCL) | LOT XXXX EXP XXXX 250/500 Diluent B (DFL) | LOT XXXX EXP XXXX 3200/3500 Hemolytic Agent A (WNR) | LOT XXXX EXP XXXX 200/3500 Hemolytic Agent B (WDF) | LOT XXXX EXP XXXX 0/500 RESERVE TANK MODE 80 TESTS REMAINING Hemolytic Agent C (SLS) |

D304a    D304a

D303

D201    XR-1000

Reagent replacement is necessary.

D202
D200

D1
D100
D300a

EP 4 782 842 A1

63

## EUROPEAN SEARCH REPORT

**Application Number**

EP 26 15 3380

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2024/302391 A1 (MISAWA KOTA [JP] ET AL) 12 September 2024 (2024-09-12) * the whole document * | 1-17 | INV. G01N35/10 |
| A | EP 2 410 340 A1 (SYSMEX CORP [JP]) 25 January 2012 (2012-01-25) * the whole document * | 1-17 | |
| A | CN 111 562 402 A (SHANGHAI HUIZHONG MEDICAL SCIENCE AND TECH CO LTD ET AL.) 21 August 2020 (2020-08-21) * the whole document * | 1-17 | |
| A,D | WO 2007/047069 A2 (BECKMAN COULTER INC [US]) 26 April 2007 (2007-04-26) * the whole document * | 1-17 | |
| A | EP 4 246 120 A1 (SYSMEX CORP [JP]) 20 September 2023 (2023-09-20) * the whole document * | 1-17 | |
| A | H. TAILOR ET AL: "Evaluation of the Sysmex XN-550, a Novel Compact Haematology analyser from the XN-L series, compared to the XN-20 system", INTERNATIONAL JOURNAL OF LABORATORY HEMATOLOGY WILEY-BLACKWELL PUBLISHING LTD., GB; US, vol. 39, no. 6, 25 June 2017 (2017-06-25), pages 585-589, XP072386577, ISSN: 1751-5521, DOI: 10.1111/IJLH.12701 * the whole document * | 1-17 | **TECHNICAL FIELDS SEARCHED (IPC)** G01N B01L |

The present search report has been drawn up for all claims

| Place of search | Date of completion of the search | Examiner |
|---|---|---|
| The Hague | 18 June 2026 | Dekker, Derk |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 26 15 3380

This annex lists the patent family members relating to the patent documents cited in the above-mentioned European search report.
The members are as contained in the European Patent Office EDP file on
The European Patent Office is in no way liable for these particulars which are merely given for the purpose of information.

18-06-2026

| Patent document cited in search report | | Publication date | Patent family member(s) | | Publication date |
|---|---|---|---|---|---|
| US 2024302391 | A1 | 12-09-2024 | CN | 118624920 A | 10-09-2024 |
| | | | EP | 4428540 A1 | 11-09-2024 |
| | | | JP | 2024127094 A | 20-09-2024 |
| | | | US | 2024302391 A1 | 12-09-2024 |
| EP 2410340 | A1 | 25-01-2012 | CN | 102356318 A | 15-02-2012 |
| | | | EP | 2410340 A1 | 25-01-2012 |
| | | | EP | 3543706 A1 | 25-09-2019 |
| | | | JP | 5679963 B2 | 04-03-2015 |
| | | | JP | 5710043 B2 | 30-04-2015 |
| | | | JP | 5800963 B2 | 28-10-2015 |
| | | | JP | 2014139580 A | 31-07-2014 |
| | | | JP | 2014238408 A | 18-12-2014 |
| | | | JP | WO2010107042 A1 | 20-09-2012 |
| | | | US | 2012003121 A1 | 05-01-2012 |
| | | | US | 2016131674 A1 | 12-05-2016 |
| | | | WO | 2010107042 A1 | 23-09-2010 |
| CN 111562402 | A | 21-08-2020 | NONE | | |
| WO 2007047069 | A2 | 26-04-2007 | EP | 1933982 A2 | 25-06-2008 |
| | | | JP | 5246778 B2 | 24-07-2013 |
| | | | JP | 2009511818 A | 19-03-2009 |
| | | | US | 2007086923 A1 | 19-04-2007 |
| | | | WO | 2007047069 A2 | 26-04-2007 |
| EP 4246120 | A1 | 20-09-2023 | BR | 102023003038 A2 | 02-01-2024 |
| | | | EP | 4246120 A1 | 20-09-2023 |
| | | | EP | 4246121 A1 | 20-09-2023 |
| | | | US | 2023296522 A1 | 21-09-2023 |
| | | | US | 2023341314 A1 | 26-10-2023 |
| | | | US | 2025389663 A1 | 25-12-2025 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2025010999 A

- WO 2007047069 A